# EUROPÄISCHE PATENTANMELDUNG

(11) **EP 2 485 009 A1**
(43) Veröffentlichungstag der Anmeldung: **08.08.2012**
(21) Anmeldenummer: 11405289.7
(22) Anmeldetag: 18.07.2011
(51) Int. Cl.: G01B 9/02, A61B 3/10

(54) **Frequenzbereichs-OCT**

(30) Priorität: 04.02.2011 CH 2102011
(71) Anmelder: HAAG-STREIT AG, CH-3098 Köniz (CH)
(72) Erfinder: Wälti, Rudolf, 3150 Schwarzenburg (CH); Breitenstein, Jörg, 3052 Zollikofen (CH)
(74) Vertreter: Stäbler, Roman

(57) **Zusammenfassung**

Eine Vorrichtung zur Ermittlung geometrischer Werte wenigstens eines ersten Bereichs (MB1) und eines vom ersten Bereich (MB1) distanzierten zweiten Bereichs (MB3) eines transparenten oder diffusiven Gegenstands, umfasst einen Kohärenztomographen mit einem Objektarm, einem Referenzarm, einem Detektorarm und einer Lichtquelle (ALQ) zum emittieren von Licht. Die Vorrichtung weist einen durch den Objektarm und/oder den Referenzarm gebildeten ersten Weg einer ersten optischen Weglänge und einen zweiten Weg einer zweiten optischen Weglänge auf, entlang welcher sich das von der Lichtquelle (ALQ) emittierte Licht fortpflanzen kann.

## Beschreibung

### Technisches Gebiet

Die Erfindung betrifft eine Vorrichtung und ein Verfahren zur Ermittlung geometrischer Werte wenigstens eines ersten Bereichs und eines vom ersten Bereich distanzierten zweiten Bereichs eines transparenten oder diffusiven Gegenstands, umfassend einen Kohärenztomographen mit einem Objektarm, einem Referenzarm, einem Detektorarm und einer Lichtquelle zum emittieren von Licht.

### Stand der Technik

Die vorliegende Erfindung betrifft ein Verfahren und eine Vorrichtung zur Ermittlung geometrischer Werte wenigstens zweier voneinander distanzierter Bereiche bei einem transparenten oder diffusiven Gegenstand, insbesondere zur Ermittlung von Schichtdicken und Längen und / oder Flächenkrümmungen (Topographie) als geometrische Werte. Unter geometrischen Werten werden beispielsweise Schichtdicken, Abstände, Längen und Topographien verstanden.

Das Grundprinzip der OCT basiert auf der Interferometrie. Dieses Verfahren vergleicht die Laufzeit eines Signals mit Hilfe eines Interferometers (meist Michelson-Interferometer). Dabei wird der eine Arm mit bekannter optischer Weglänge als Referenzarm zum Messarm herangezogen.

Die Interferenz der Signale (optische Kreuzkorrelation) aus beiden Armen ergibt ein Muster, aus dem man die relative optische Weglänge innerhalb eines Tiefenprofils (amplitude-mode scan) herauslesen kann. In den eindimensionalen Rasterverfahren wird der Strahl dann transversal in einer oder zwei Richtungen geführt, womit ein flächiges Tomogramm (brightness-mode scan) oder eine dreidimensionale Topographie (c-mode scan) aufgenommen werden kann.

OCT ist insbesondere in der Augenheilkunde stark verbreitet, was unter anderem darauf zurückzuführen ist, dass die Tiefenauflösung von der transversalen Auflösung entkoppelt ist und dass sie berührungslose in vivo Messungen erlaubt. Durch die geringe notwendige Leistung bei der Messung bilden sich weitere Vorteile bei lichtempfindlichen Körpern, wie z.B. bei Messungen am Auge.

Die bekannten Vorrichtungen zur Ermittlung von geometrischen Werten haben den Nachteil, dass sie relativ niedrige Messgeschwindigkeiten und Siganl-Rausch-Verhältnisse aufweisen. Zudem ist der Aufbau relativ komplex und damit teuer. Schliesslich sind häufig die Messbereiche nicht zufriedenstellend.

### Darstellung der Erfindung

Aufgabe der Erfindung ist es, eine dem eingangs genannten technischen Gebiet zugehörende Vorrichtung zur Ermittlung geometrischer Werte mittels eines Kohärenztomographen zu schaffen, welche sich gegenüber den bekannten Geräten durch höhere Messgeschwindigkeiten und einen grösseren Messbereich auszeichnet.

Die Lösung der Aufgabe ist durch die Merkmale des Anspruchs 1 definiert. Gemäss der Erfindung weist die Vorrichtung einen durch den Objektarm und/oder den Referenzarm gebildeten ersten Weg einer ersten optischen Weglänge und einen zweiten Weg einer zweiten optischen Weglänge auf, entlang welcher sich das von der Lichtquelle emittierte Licht fortpflanzen kann.

Es gilt, dass die Kohärenzlänge der durchstimmbaren Laserlinie der Messtiefe im Objekt entspricht. Weil die Kohärenzlänge der durchstimmbaren Lichtquelle begrenzt ist, ist auch die Messtiefe begrenzt. Die menschliche Achslänge von maximal ca. 34 mm ist länger als die Kohärenzlänge der meisten heute kommerziell erhältlichen abstimmbaren Lichtquellen. Deshalb kann eine Achslänge von 34 mm von den meisten heute erhältlichen abstimmbaren Lichtquellen ohne die Verwendung von zusätzlichen Massnahmen nicht gemessen werden. Erfindungsgemäss wird nun eine Massnahme vorgeschlagen, welche erlaubt, Achslängen von maximal ca. 34 mm zu messen, nämlich die Unterteilung des Messbereichs in zwei voneinander distanzierte Messbereiche (Tiefenscanbereiche), welche jeweils dem obig erwähnten erste Weg, respektive dem zweiten Weg entsprechen. Natürlich können auch mehr als zwei voneinander distanzierte Messbereiche vorgesehen sein. Die zwei voneinander distanzierten Bereiche werden im Folgenden als vorderer und hinterer Messbereich bezeichnet.

Der erste Weg der ersten optischen Weglänge und der zweite Weg der zweiten optischen Weglänge können auf verschiedene Arten erreicht werden. So kann zum Beispiel auf genau einem optischen Arm (Referenzarm oder Objektarm) ein verfahrbarer Spiegel vorgesehen sein. Durch verschieben des Spiegels kann damit von einer ersten Weglänge auf eine zweite Weglänge umgeschaltet werden. Da je nach Ausführungsform der Spiegel auch mehr als zwei Positionen anfahren kann, könnten grundsätzlich auch mehrere, optische Weglängen, insbesondere bei einer kontinuierlichen Einstellungsmöglichkeit, beliebig viele optische Weglängen eingestellt werden. In weiteren Ausführungsformen kann der Objektoder Referenzarm auch einen ein- und ausschwenkbaren Spiegel umfassen, womit die beiden Weglängen eingestellt werden können. Dem Fachmann sind dazu auch weitere Möglichkeiten bekannt.

Weiter können die beiden optischen Weglänge auch separat, durch jeweils einen eigenen optischen Arm, bereitgestellt sein.

Um das Licht von einem ersten Arm zu einem zweiten Arm umzulenken ist vorzugsweise ein Scannerspiegel vorgesehen, welcher in einer ersten Position den Lichtstrahl in den ersten Arm und in einer zweiten Position den Lichtstrahl in den zweiten Arm leiten kann. Weiter kann das Licht auch mit einem Scannerspiegel, insbesondere einem Galvanometerspiegel, einem faseroptischen Schalter oder einem Flüssigkristall vom einen zum anderen Arm gelenkt werden. Um Dispersionen zu kompensieren kann in einem Referenzarm ein Glassubstrat vorgesehen sein. Damit kann das Interferenzsignal der Retina erhöht werden. In Varianten kann auf einen Dispersionskompensator auch verzichtet werden.

Vorzugsweise umfasst die Vorrichtung einen Fokusschalter im Objektarm. Damit können die beiden Bereiche fokussiert werden. Falls das Licht nacheinander zwei Arme durchläuft, erfolgt die Umschaltung des Fokus vorzugsweise synchron mit der Umschaltung vom ersten Weg der ersten optischen Weglänge zum zweiten Weg der zweiten optischen Weglänge. Der Fokusschalter kann auch als einschwenkbares optisches Element ausgebildet sein. Auch dazu sind dem Fachmann weitere Möglichkeiten bekannt, so zum Beispiel eine Linse, welche je nach Orientierung andere Brennweiten aufweist.

Die Vorrichtung kann weiter eine Kamera aufweisen, welche über einen Wellenlängenselektiven Strahlteiler mit sichtbarem Licht gespiesen werden kann. Dies hat den Vorteil, dass die für die OCT-Messung notwendigen Wellenlängen (z.B. infrarot) nicht oder nur unwesentlich abgeschwächt wird. Diese Kamera wird vorzugsweise im Bereich des zu vermessenden Objekts platziert. Damit kann eine Vorderseite des Objektes, insbesondere des Auges aufgenommen und an einem Bildschirm dargestellt werden. Ein Benutzer kann so zum Beispiel mittels eines Kreutzschlittens das Messgerät positionieren.

Die Vorrichtung kann weiter ein optisches Element zum Projizieren eines Musters auf das Objekt umfassen. Dieses optische Element kann z.B. als Konus oder Halbkugel ausgebildet und das Muster kann als Ringmuster vorgesehen sein, welches mittels einer Kamera aufgenommen werden kann. Damit kann z.B. bei einem Auge eine Oberflächenform des Tränenfilms errechnet werden, um mit diesen ermittelten Daten wiederum die Messgenauigkeit der OCT-Messung zu optimieren.

Typischerweise werden für den vorderen Bereich und den hinteren Bereich Licht derselben Wellenlänge verwendet. In Varianten können für den vorderen Bereich und den hinteren Bereich Licht unterschiedlicher Wellenlänge eingesetzt werden. Damit kann je nach Empfindlichkeit der Bereiche des Gegenstandes, insbesondere des Auges, die Leistung angepasst werden, womit eine Sensitivität erhöht werden kann. Anderseits kann diese Ausgestaltung die Nachteile aufweisen, dass die Vorrichtung teurer und aufwendiger in der Konstruktion wird. Dazu kann die Vorrichtung wellenlängenselektive Strahlteiler und gegebenenfalls mehrere Lichtquellen umfassen.

Im Verfahren zur Ermittlung geometrischer Werte wenigstens eines ersten Bereichs und eines vom ersten Bereich distanzierten zweiten Bereichs eines transparenten oder diffusiven Gegenstands, wird ein Kohärenztomograph eingesetzt, welcher einen Objektarm, einen Referenzarm, einen Detektorarm und eine Lichtquelle zum Emittieren von Licht umfasst. Zur Ermittlung des geometrischen Wertes des ersten Bereichs wird das Licht der Lichtquelle über einen ersten Weg einer ersten optischen Weglänge des Objektarms und/oder des Referenzarms geführt. Zur Ermittlung des geometrischen Wertes des zweiten Bereichs wird das Licht der Lichtquelle über einen zweiten Weg einer zweiten optischen Weglänge des Objektarms und/oder des Referenzarms geführt.

Vorzugsweise ist der Kohärenztomograph als Frequenzbereichs-OCT, insbesondere als SSOCT (swept source OCT) oder als spektraler OCT, ausgebildet.

Vorzugsweise wird also ein optischer Kohärenztomograph im Frequenzbereich z.B. in der geometrischen Ausführung eines Michelson-Interferometers verwendet. Diese

Interferenzmethode wird Frequenzbereichs-OCT (englisch: Frequency Domain OCT, wobei OCT für Optical Coherence Tomograph steht) genannt. Sie hat im Gegensatz zu der schon länger existierenden Zeitbereichs-OCT (englisch: Time-Domain OCT) die Eigenschaft, dass eine Tiefenmessung ohne beweglichen Referenzarm möglich ist, und dass die Tiefenzuordnung der vom Messobjekt reflektierten Signale über eine Schwebungsfrequenz erfolgt.

Die Frequenzbereichs-OCT existiert in zwei Varianten:
1. Eine Variante besteht darin, eine abstimmbare Lichtquelle zu verwenden, die ihre Wellenlänge periodisch verändert (englisch: Swept Source OCT, SSOCT oder Wavelength-Tuning OCT). Die abstimmbare Lichtquelle emittiert eine schmale Spektrallinie (Laserlinie), die mit einer bestimmten zeitlichen Periode durch einen Abstimmbereich hin- und hergeschoben wird. Dabei ist der Messbereich in die Tiefe eines Messobjekts gegeben durch die Linienbreite der durchstimmbaren Laserlinie. Die Repetitionsrate der Messungen durch ein Messobjekt ist gegeben durch die zeitliche Periode der Durchstimmung. Das zeitliche Schwebungssignal, das durch Interferenz der aus dem Referenzarm und Objektarm reflektierten Laserlinie entsteht, kann mit einer Fotodiode einmal pro Durchstimmperiode detektiert werden.
2. Die andere Methode heisst spektrale OCT, bei der eine Lichtquelle mit zeitlich unveränderlichem Spektrum verwendet wird. Für die Detektion ist ein Spektrometer notwendig, um das spektrale Schwebungssignal wellenlängenabhängig aufzunehmen. Dabei ist der Messbereich in die Tiefe gegeben durch die Auflösung des Spektrometers. Die Repetitionsrate der Messungen durch das Messobjekt ist gegeben durch die Auslesegeschwindigkeit des im Spektrometer verwendeten Zeilendetektors.

Weil bei diesen beiden Frequenzbereichsverfahren das vom Messobjekt und vom Referenzarm zurück gestreute Licht ein Schwebungssignal mit zur Tiefe proportionaler Frequenz erzeugt, können durch eine Fouriertransformation die Streuamplituden bei jeder Tiefe berechnet werden. Die Frequenzbereichs-OCT ermöglicht höhere Messgeschwindigkeiten und ein besseres Signal-Rausch-Verhältnis als die Zeitbereichs-OCT. Die Frequenzbereichs-OCT hat jedoch den Nachteil einer mit der Messtiefe abnehmenden Signalamplitude.

Um ausgeprägte Interferenzsignale auch bei nur schwach reflektierenden Schichten im Objektarm zu erhalten, wird bevorzugt die Messstrahlung zeitlich nacheinander im vorderen und im hinteren Messbereich fokussiert. Die Verschiebung des Fokus vom vorderen in den hinteren Messbereich geschieht synchron, zum Beispiel mit dem Wechsel des für die Messung verwendeten Referenzarms, wenn zwei Referenzarme verwendet werden, oder synchron mit dem Sprung in der optischen Weglänge des Referenzarms, wenn nur ein Referenzarm verwendet wird. Dem Fachmann sind auch weitere Möglichkeiten bekannt.

Vorzugsweise ist der geometrische Wert eine Schichtdicke, eine Länge, eine Flächenkrümmung und/oder eine Topographie des Gegenstandes. Die Vorrichtung zur Ermittlung von Schichtdicken und Längen und / oder Flächenkrümmungen (Topographie) als geometrische Werte. Unter geometrischen Werten werden beispielsweise Schichtdicken, Abstände, Längen und Topographien verstanden. Prinzipiell kann also ein geometrischer Wert ein Punkt oder Vektor in einem vorzugsweise dreidimensionalen, z. B. kartesischen Koordinatensystem sein. Der Punkt, respektive Vektor kann auch eine höhere Dimension aufweisen, wobei eine Komponente des Vektors z.B. eine Wellenlänge, eine Polarisation etc. sein kann. Natürlich kann der geometrische Wert auch eine Vielzahl von Punkten, Vektoren, Schichtdicken, Längen, Flächenkrümmungen und/oder Topographien des Gegenstandes umfassen.Dem Fachmann sind auch weitere geometrische Werte bekannt, welche mittels dieser Vorrichtung ermittelt werden können.

Vorzugsweise umfasst der Objektarm einen Fokusschalter. Falls die Vorrichtung zwei Objektarme umfasst, wobei sich das Licht abwechselnd in diesen Objektarmen fortpflanzt, können die Foki durch eine geeignete Linsenwahl erreicht werden. Falls jedoch in einem Objektarm zwei unterschiedliche optische Weglängen erreicht werden sollen, kann der Fokusschalter als Flüssiglinse oder Flüssigkristall ausgebildet sein.

In Varianten kann auf den Fokusschalter auch verzichtet werden, insbesondere wenn Gegenstände vermessen werden, bei welchen eine Änderung des Fokus nicht notwendig ist.

Bevorzugt sind der erste Bereich ein vorderer Bereich eines Auges, insbesondere die Vorderseite der Kornea, und der zweite Bereich ein hinterer Bereich des Auges, insbesondere die Retina.

Dem Fachmann ist allerdings klar, dass auch andere Objekte als Augen (Haut, oder reflektierende Körper im Allgemeinen), respektive andere Bereiche des Auges, insbesondere z.B. der Glaskörper des Auges im Allgemeinen etc. untersucht werden können.

In den nachfolgenden Absätzen sind anhand fünf Arten (1. Art bis 5. Art) beschrieben, wie die unterschiedlichen Tiefenscanbereiche erzeugt werden können.

### 1. Art

Vorzugsweise sind der erste Weg mit der ersten optischen Weglänge durch einen ersten Objektarm und der zweite Weg mit der zweiten optischen Weglänge durch einen zweiten Objektarm gegeben. Dabei umfasst die Vorrichtung in dieser Ausführungsform insbesondere genau einen Referenzarm.

Dem Fachmann ist klar, dass die Vorrichtung auch mehr als zwei, z.B. drei Objektarme umfassen kann.

Im entsprechenden Verfahren wird dabei bevorzugt das Licht nacheinander in den ersten Objektarm mit dem ersten Weg der ersten optischen Weglänge und in den zweiten Objektarm mit dem zweiten Weg der zweiten optischen Weglänge geführt. Das Licht kann zum Beispiel abwechslungsweise, d.h. alternierend, in die beiden Arme geleitet werden.

Das Licht der Lichtquelle pflanzt sich also abwechslungsweise in zwei unterschiedlich langen Objektarmen fort, wobei vorzugsweise ein Referenzarm verwendet wird. Der Unterschied der optischen Länge der zwei Objektarme entspricht der optischen Distanz zwischen den zwei voneinander distanzierten Bereichen.

Die optischen Arme können Polarisationskontroller aufweisen, womit die Polarisation des Lichtes der Referenzarme an die Polarisation des Lichtes des Objektarms angeglichen werden kann.

Der Referenz- und Objektarm kann weiter ein drehbares Element umfassen, das aus einer Drehachse, einer halbkreisförmigen Glasplatte, einem halbkreisförmigen Absorber und einem halbkreisförmigen Loch besteht. Das drehbare Element kann abwechslungsweise während je einer halben Umdrehung einen ersten und einen zweiten optischen Arm freischalten, indem der jeweilige Lichtstrahl entweder vom absorbierenden Material des drehbaren Elements absorbiert oder durch das Loch des drehbaren Elements transmittiert wird.

Vorzugsweise wird der Fokus synchron mit der Messdistanz verschoben. Die Objektarme unterscheiden sich in der Brechkraft deren Optiken sowie in deren Längen. Ein X- und ein Y-Scanner werden vorzugsweise durch die Objektarme gemeinsam verwendet, womit eine effiziente, kostengünstige und einfach aufgebaute Vorrichtung erreicht wird. Der X- und der Y-Scanner können durch zwei separate Scanner, aber auch durch einen einzigen Scanner realisiert sein.

### 2. Art

In einer weiteren bevorzugten Ausführungsform ist die erste optische Weglänge durch einen ersten Referenzarm und die zweite optische Weglänge durch einen zweiten Referenzarm gegeben.

Im entsprechenden Verfahren wird das Licht vorzugsweise nacheinander in einem ersten Referenzarm mit dem ersten Weg der ersten optischen Weglänge und in einem zweiten Referenzarm mit dem zweiten Weg der zweiten optischen Weglänge geführt, wobei das Licht wiederum zum Beispiel abwechslungsweise in die beiden Referenzarme geleitet werden kann.

Das Licht der Lichtquelle pflanzt sich also abwechslungsweise in zwei unterschiedlich langen Referenzarmen fort, wobei vorzugsweise ein Objektarm verwendet wird. Der

Unterschied der optischen Länge der zwei Referenzarme entspricht der optischen Distanz zwischen den zwei voneinander distanzierten Bereichen.

### 3. Art

In einer weiteren bevorzugten Ausführungsform ist die erste optische Weglänge durch einen ersten Referenzarm und die zweite optische Weglänge durch einen ersten Objektarm und eine dritte optische Weglänge durch einen zweiten Referenzarm und eine vierte optische Weglänge durch einen zweiten Objektarm gegeben. In diesem Fall werden vorzugsweise jeweils ein Referenzarm und ein Objektarm als paar verwendet.

Im entsprechenden Verfahren ist das Licht bevorzugt in einem ersten Referenzarm mit dem ersten Weg der ersten optischen Weglänge und in einem ersten Objektarm mit dem zweiten Weg der zweiten optischen Weglänge, sowie anschliessend in einem zweiten Referenzarm mit einem dritten Weg der dritten optischen Weglänge und einem zweiten Objektarm mit einem vierten Weg der vierten optischen Weglänge geführt. Somit bilden der erste Referenzarm und der erste Objektarm ein Paar, in welchen nacheinander das Licht geführt ist. Im Anschluss kann das Licht in einem zweiten Paar optischer Arme, nämlich im zweiten Objektarm und zweiten Referenzarm, geführt sein.

In einer bevorzugten Ausführungsform kann ein drehbarer Spiegel sowohl als Distanz- als auch als Fokusschalter wirken, indem mit diesem Spiegel der Referenzstrahl und der Objektstrahl beeinflusst wird. Damit wird ein besonders einfacher und kompakter Aufbau der Vorrichtung erreicht.

### 4. Art

Vorzugsweise umfasst die Vorrichtung einen Objektarm oder einen Referenzarm mit einem einschwenkbaren und ausschwenkbaren optischen Element, wobei die erste optische Weglänge bei eingeschwenktem optischem Element gegeben ist und die zweite optische Weglänge bei ausgeschwenktem optischem Element gegeben ist. In diesem Fall kann der Fokusschalter zum Beispiel als Flüssiglinse oder als Flüssigkristall ausgebildet sein.

Im entsprechenden Verfahren wird im Objektarm oder im Referenzarm vorzugsweise ein optisches Element eingeschwenkt und ausgeschwenkt, so dass sich bei eingeschwenktem optischen Element ein erster Weg der ersten optischen Weglänge und bei ausgeschwenktem optischen Element ein zweiter Weg der zweiten optischen Weglänge einstellt, wobei das Licht nacheinander, insbesondere abwechselnd, im ersten Weg und im zweiten Weg geführt ist. Das optische Element kann z.B. als Spiegel ausgebildet sein.

Das Licht der Lichtquelle wird also in einen einzigen Arm (ein Referenzarm oder ein Objektarm) geleitet, der zeitlich nacheinander zwei unterschiedliche Weglängen aufweist. Die optische Änderung der Armlänge entspricht der optischen Distanz zwischen den zwei voneinander distanzierten Bereichen.

Das optische Element kann dabei aus einem Prisma und einer Glasplatte ausgebildet sein, womit synchron der Fokus und der Messbereich zwischen dem vorderen Augensegment und dem hinteren Augensegment hin- und hergeschaltet werden kann.

Auch in dieser Ausführungsform kann der Referenzarm und Objektarm ein drehbares Element umfassen, das aus einer Drehachse, einer halbkreisförmigen Glasplatte, einem halbkreisförmigen Absorber und einem halbkreisförmigen Loch besteht. Das drehbare Element kann abwechslungsweise während je einer halben Umdrehung einen ersten und einen zweiten optischen Arm freischalten, indem der jeweilige Lichtstrahl entweder vom absorbierenden Material des drehbaren Elements absorbiert oder durch das Loch des drehbaren Elements transmittiert wird. Zudem kann das drehbare Element während einer halben Umdrehung eine Glasplatte in den Strahlengang des Objektarms einführen.

### 5. Art

Die Vorrichtung weist vorzugsweise einen ersten Arm mit einer ersten optischen Weglänge und einen zweiten Arm mit einer zweiten optischen Weglänge auf, wobei der erste und der zweite Arm jeweils als Objektarm oder Referenzarm ausgebildet sind, und wobei ein Arm ein optisches Transformationselement zur Selektion einer Eigenschaft des Lichtes, insbesondere der Wellenlänge oder der Polarisation, umfasst und wobei der Detektorarm eine optische Trenneinrichtung welche dem optischen Transformationselement entspricht, umfasst.

Im entsprechenden Verfahren wird das Licht vorzugsweise gleichzeitig in zwei Arme, insbesondere einem Objektarm, respektive Referenzarm, unterschiedlicher optischer Weglänge geleitet, wobei eine optische Eigenschaft des Lichts, insbesondere die Polarisation oder die Wellenlänge, in einem ersten Arm sich von derselben optischen Eigenschaft des zweiten Arms unterscheidet und wobei im Detektorarm das Licht anhand derselben optischen Eigenschaft mittels einer optischen Trenneinrichtung getrennt werden.

Das Licht der Lichtquelle wird also gleichzeitig in zwei unterschiedlich lange Arme geleitet, wobei das Licht des einen Arms sich vom Licht des anderen Arms in einer bestimmten Eigenschaft unterscheidet (insbesondere in der Polarisation oder Wellenlänge). In diesem Fall wird mit einer geeigneten Trenneinrichtung erreicht, dass das Licht mit unterschiedlicher Eigenschaft unterschiedlich lange Wege im Referenz- oder Objektarm durchläuft. Zudem wird mit einer geeigneten Trenneinrichtung im Detektionsarm erreicht, dass die zwei Interferenzen auf unterschiedliche Detektoren geleitet werden.

Um die Polarisation kontrollieren zu können, kann ein polarisierender Strahlenteilerwürfel im Strahlengang vorgesehen sein.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine 1. Ausführungsvariante der Vorrichtung zur Ermittlung geometrischer Werte der zweiten Art mit zwei Referenzarmen;
- Fig. 2: eine 2. Ausführungsvariante der Vorrichtung zur Ermittlung geometrischer Werte der zweiten Art mit zwei Referenzarmen;
- Fig. 3: eine 3. Ausführungsvariante der Vorrichtung zur Ermittlung geometrischer Werte der vierten Art mit einem einzigen Referenzarm, welcher zeitlich nacheinander unterschiedliche Weglängen aufweist;
- Fig. 4: eine 4. Ausführungsvariante der Vorrichtung zur Ermittlung geometrischer Werte der zweiten Art mit zwei Referenzarmen, basierend auf spektraler OCT;
- Fig. 5: eine 5. Ausführungsvariante der Vorrichtung zur Ermittlung geometrischer Werte der zweiten Art mit zwei Referenzarmen, basierend auf SSOCT;
- Fig. 6: eine 6. Ausführungsvariante der Vorrichtung zur Ermittlung geometrischer Werte der zweiten Art mit zwei Referenzarmen und drei Flüssiglinsen;
- Fig. 7: eine 7. Ausführungsvariante der Vorrichtung zur Ermittlung geometrischer Werte der zweiten Art mit zwei Referenzarmen und zwei Flüssigkristallen;
- Fig. 8a, 8b: eine 8. Ausführungsvariante der Vorrichtung zur Ermittlung geometrischer Werte der ersten Art mit zwei Objektarmen und einem Spiegel-Schalter;
- Fig. 9a, 9b: eine 9. Ausführungsvariante der Vorrichtung zur Ermittlung geometrischer Werte der ersten Art mit zwei Objektarmen;
- Fig. 10: eine 10. Ausführungsvariante der Vorrichtung zur Ermittlung geometrischer Werte der fünften Art, wobei das Licht gleichzeitig in zwei Arme geleitet wird, wobei sich das Licht der beiden Arme in der Polarisation unterscheidet;
- Fig. 11a,: 11b eine 11. Ausführungsvariante der Vorrichtung zur Ermittlung geometrischer Werte der dritten Art mit zwei Paaren eines Objekt- und Referenzarmes;
- Fig. 12a, 12b: eine 12. Ausführungsvariante der Vorrichtung zur Ermittlung geometrischer Werte der zweiten Art mit zwei Referenzarmen;
- Fig. 13a, 13b: eine 13. Ausführungsvariante der Vorrichtung zur Ermittlung geometrischer Werte der vierten Art mit einem einzigen Referenzarm, welcher zeitlich nacheinander unterschiedliche Weglängen aufweist;
- Fig. 14: eine 14. Ausführungsvariante der Vorrichtung zur Ermittlung geometrischer Werte der zweiten Art mit zwei Referenzarmen;
- Fig. 15: eine 15. Ausführungsvariante der Vorrichtung zur Ermittlung geometrischer Werte der fünften Art, wobei das Licht gleichzeitig in zwei Arme geleitet wird, wobei sich das Licht der beiden Arme in der Wellenlänge unterscheidet;
- Fig. 16: eine 16. Ausführungsvariante der Vorrichtung zur Ermittlung geometrischer Werte der fünften Art, wobei das Licht gleichzeitig in zwei Arme geleitet wird, wobei sich das Licht der beiden Arme in der Wellenlänge unterscheidet;
- Fig. 17a, 17b: eine Linse mit variabler Brennweite;
- Fig. 18a, 18b: eine schematische Darstellung der beiden durch eine umschaltbare Strahlbeschränkung erreichten Foki;
- Fig. 19a - 19c: eine 17. Ausführungsvariante der Vorrichtung zur Ermittlung geometrischer Werte der ersten Art mit zwei Objektarmen;
- Fig. 20: eine 18. Ausführungsvariante der Vorrichtung zur Ermittlung geometrischer Werte der ersten Art mit zwei Objektarmen;
- Fig. 21 a,: 21 b eine 19. Ausführungsvariante der Vorrichtung zur Ermittlung geometrischer Werte der ersten Art mit zwei Objektarmen;
- Fig. 22a-22c: eine 20. Ausführungsvariante der Vorrichtung zur Ermittlung geometrischer Werte der ersten Art mit zwei Objektarmen.

Grundsätzlich sind in den Figuren gleiche Teile mit gleichen Bezugszeichen versehen.

### Wege zur Ausführung der Erfindung

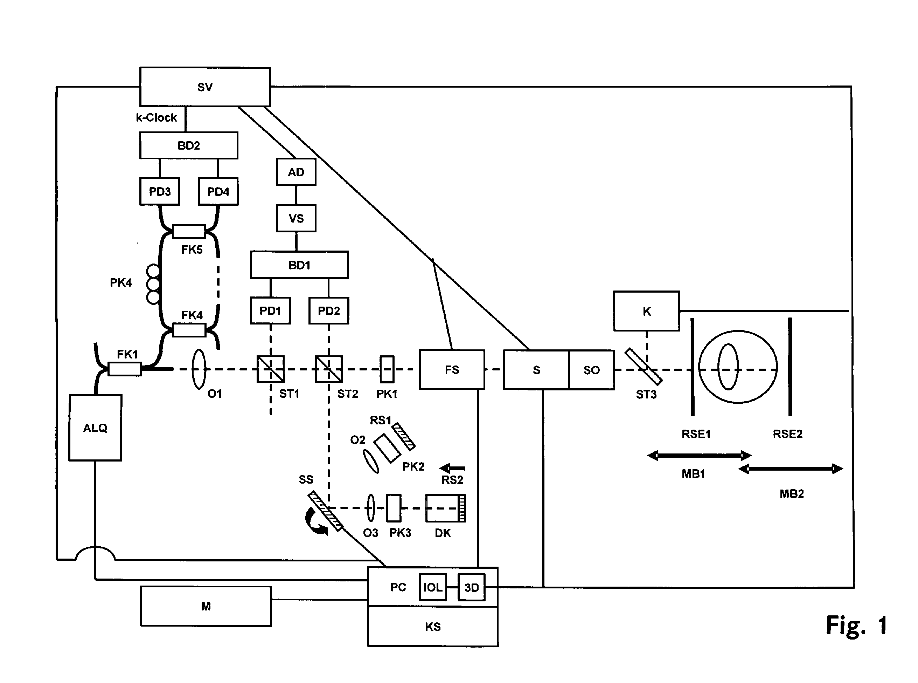

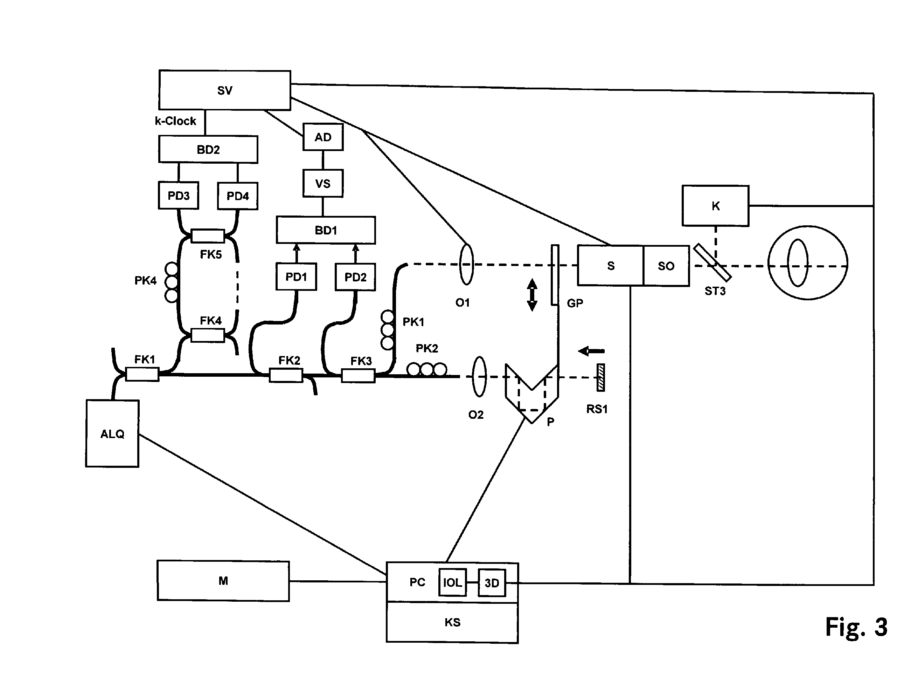

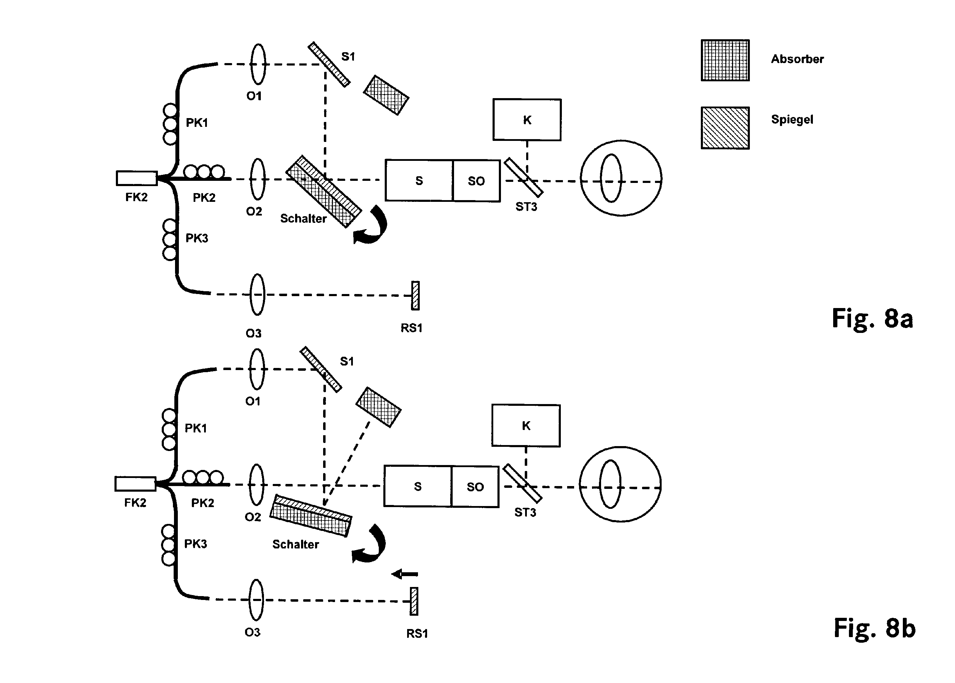

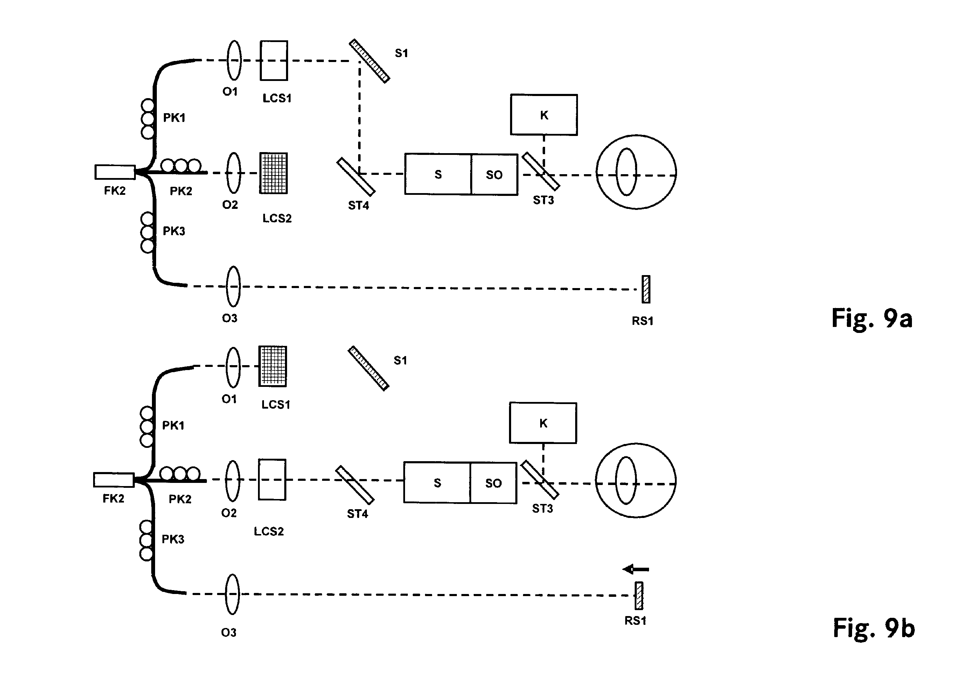

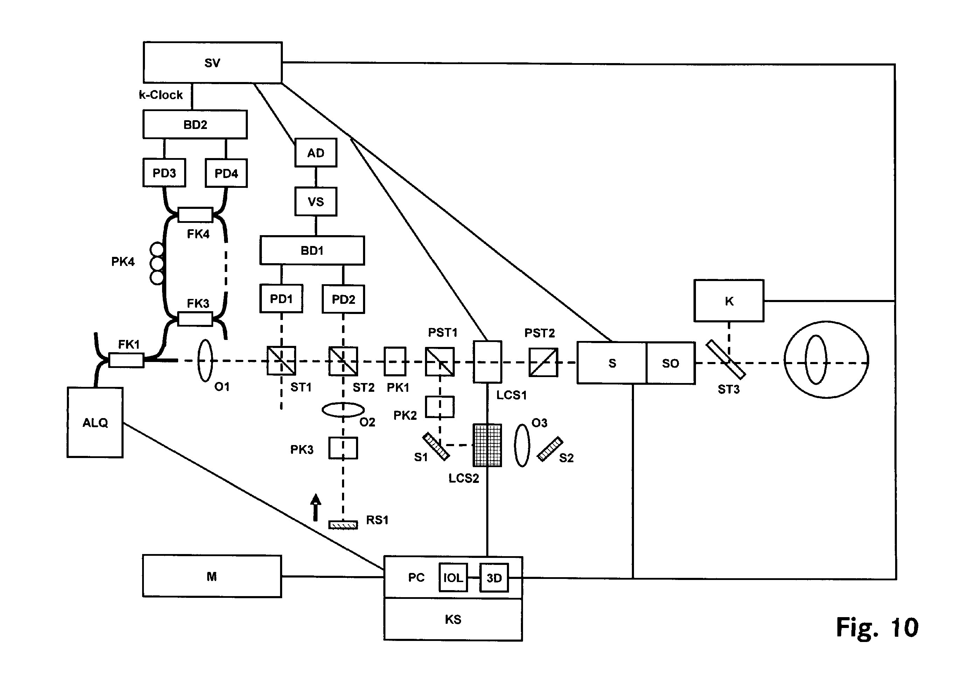

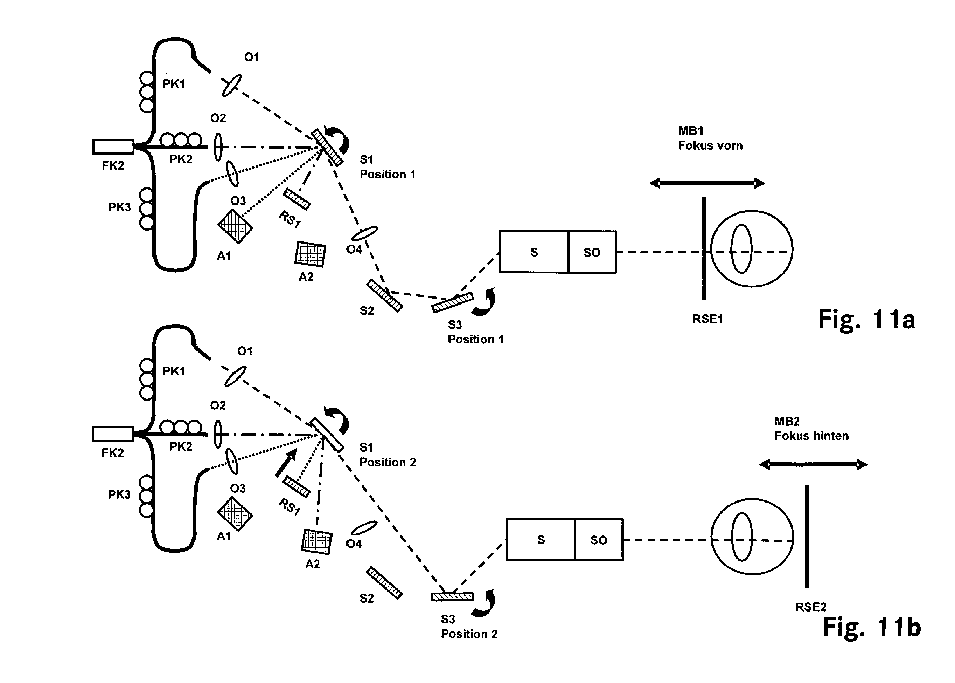

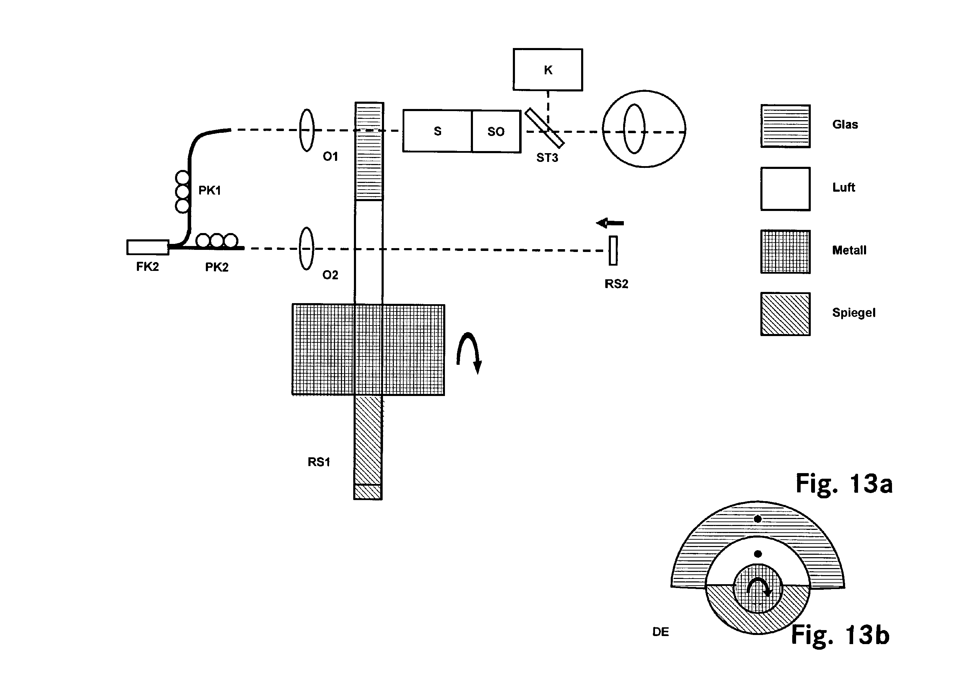

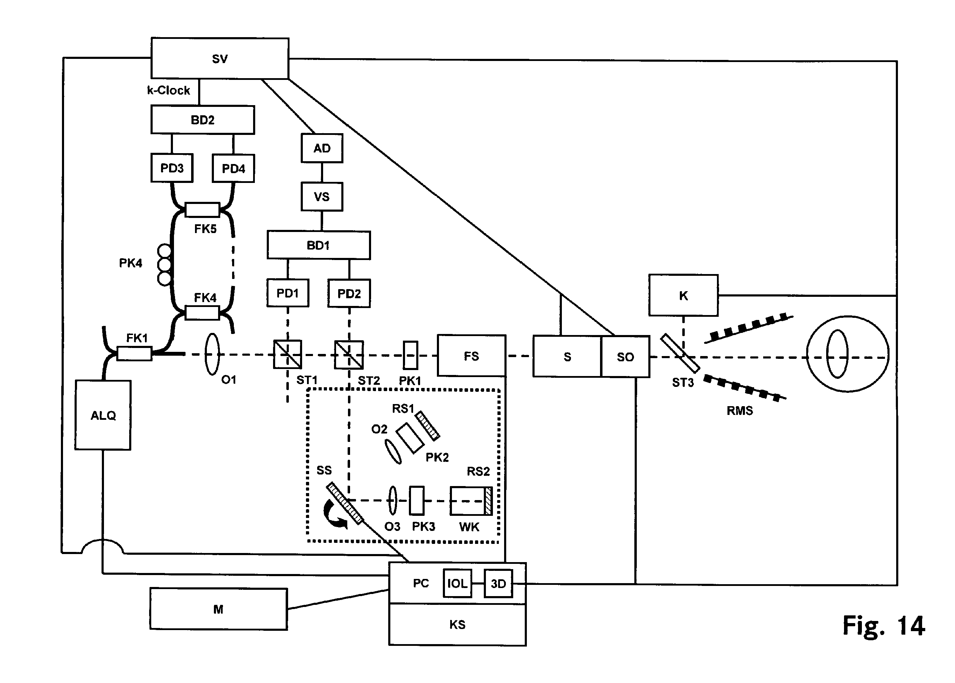

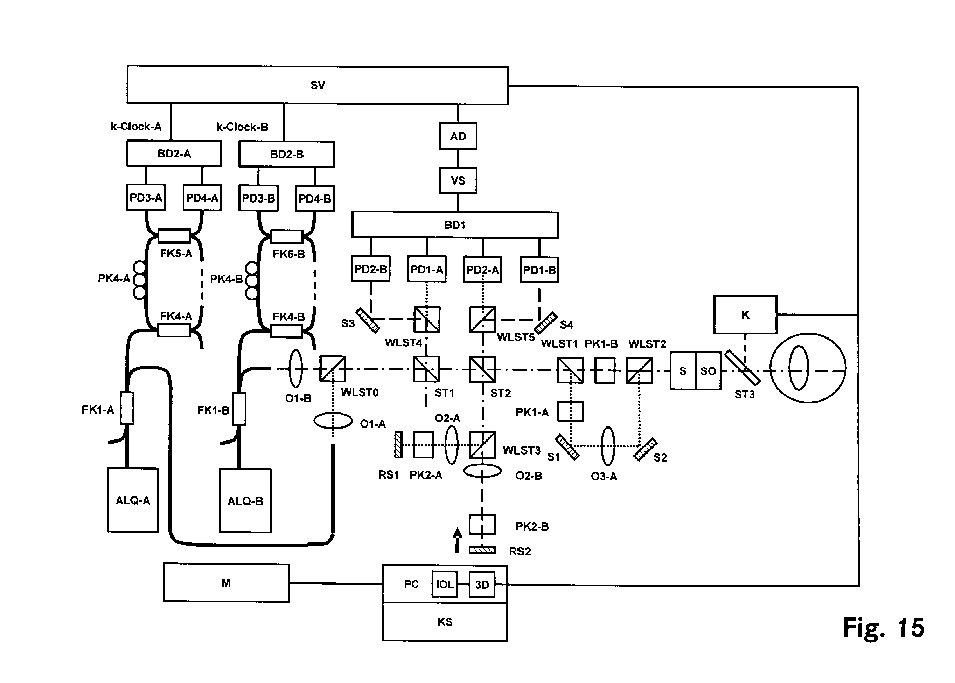

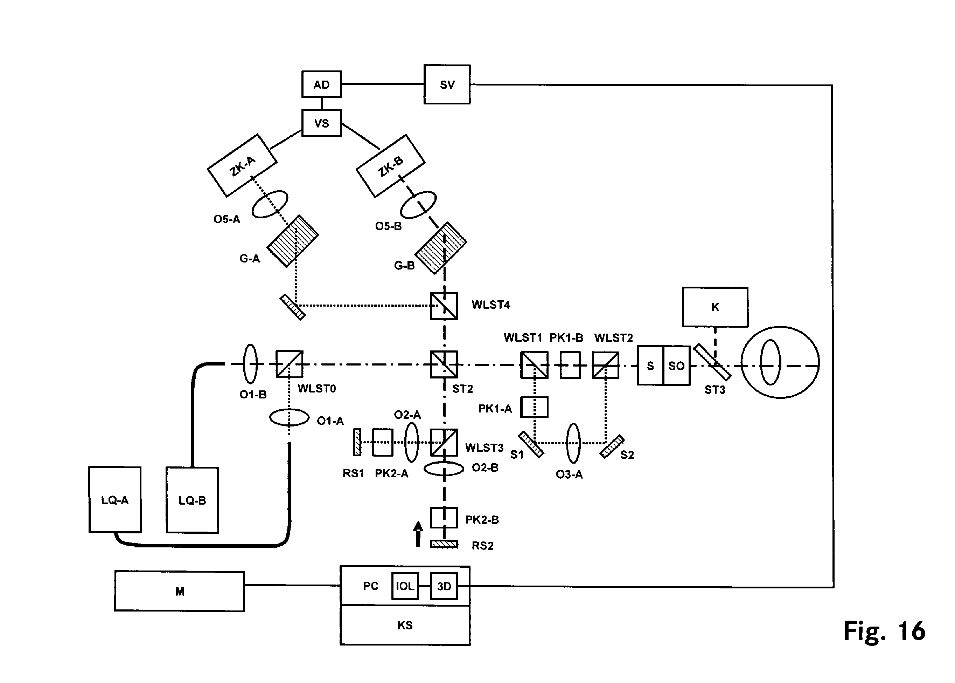

Die zwei Tiefenscanbereiche können, wie obig erwähnt, grundsätzlich auf fünf Arten erzeugt werden, welche vorgängig zwecks eines besseren Überblicks anhand der Funktionsprinzipien kurz mit Bezug auf die Figuren erläutert sind:
1. Das Licht der Lichtquelle pflanzt sich abwechslungsweise in zwei unterschiedlich langen Objektarmen fort (siehe Fig. 8a, 8b, 9a, 9b, 19a, 19b, 19c, 20, 21a, 21b, 22a, 22b, 22c), wobei ein Referenzarm verwendet wird. Der Unterschied der optischen Länge der zwei Objektarme entspricht der optischen Distanz zwischen den zwei voneinander distanzierten Bereichen.
2. Das Licht der Lichtquelle pflanzt sich abwechslungsweise in zwei unterschiedlich langen Referenzarmen fort (siehe Fig. 1, 2, 4, 5, 6, 7, 12a, 12b, 14), wobei ein Objektarm verwendet wird. Der Unterschied der optischen Länge der zwei Referenzarme entspricht der optischen Distanz zwischen den zwei voneinander distanzierten Bereichen.
3. Das Licht der Lichtquelle pflanzt sich abwechslungsweise in zwei Referenzarmen und zwei Objektarmen fort (siehe Fig. 11a, 11b), wobei je ein Referenzarm und ein Objektarm immer als Paar verwendet werden.
4. Das Licht der Lichtquelle wird in einen einzigen Arm geleitet, der zeitlich nacheinander zwei unterschiedliche Weglängen aufweist (siehe Fig. 3, 13a, 13b). Fig. 3, 13a und 13b zeigen einen Referenzarm, der zeitlich nacheinander unterschiedlich lange Weglänge aufweist. Es ist auch ein Objektarm denkbar, der zeitlich nacheinander unterschiedlich lange Weglängen aufweist. Die optische Änderung der Armlänge entspricht der optischen Distanz zwischen den zwei voneinander distanzierten Bereichen.
5. Das Licht der Lichtquelle wird gleichzeitig in zwei unterschiedlich lange Arme geleitet (siehe Fig. 9), wobei das Licht des einen Arms sich vom Licht des anderen Arms in einer bestimmten Eigenschaft unterscheidet, z.B. in der Polarisation (siehe Fig. 10) oder der Wellenlänge (siehe Fig. 15, 16). In diesem Fall muss mit einer geeigneten Trenneinrichtung dafür gesorgt werden, dass das Licht mit unterschiedlicher Eigenschaft unterschiedlich lange Wege im Referenz- oder Objektarm durchläuft. Zudem muss mit einer geeigneten Trenneinrichtung im Detektionsarm dafür gesorgt werden, dass die zwei Interferenzen auf unterschiedliche Detektoren geleitet werden.

Weil nach der durchzuführenden Fouriertransformation der gemessenen Signale aus rein mathematischen Gründen nicht entschieden werden kann, ob die optische Distanz der Reflexion im Objekt um einen Betrag z oder -z von der optischen Distanz des Referenzspiegels entfernt ist, sind die gemessenen Signale spiegelsymmetrisch um den Ort des Referenzspiegels angeordnet. Nach erfolgter Fouriertransformation gibt es also eine Hälfte von Signalen, die an der richtigen Position z erscheinen (sogenannte "Echtsignale") und eine andere Hälfte, die an einer falschen Position -z erscheinen. Die Signale, die an der falschen Stelle -z erscheinen (sogenannte "Spiegelsignale"), können nur dann identifiziert und eliminiert werden, wenn entweder die optische Distanz des Referenzarms kürzer ist als die optische Distanz der am nächsten liegenden Objektstruktur oder wenn die optische Distanz des Referenzarms länger ist als die optische Distanz der entferntesten Objektstruktur. Bei der Messung eines Objekts wie z.B. des gesamten Auges gibt es also zwei optimale Orte des Referenzspiegels. Der eine Ort entspricht also gerade der optischen Distanz bis zur Vorderseite der Kornea und der andere Ort entspricht gerade der optischen Distanz bis zur Retina. Damit die optische Distanz des Referenzspiegels bei allen Achslängen mindestens der optischen Distanz der Retina entspricht, muss sie bei der optischen Distanz der Retina der längsten zu messenden Augen (34 mm) liegen.

In Fig. 1 wird dieser Sachverhalt mit zwei vertikalen Strichen veranschaulicht. Der Strich der Referenzspiegelebene 1 RSE1 zeigt, an welchem Ort des Objektarms sich die optische Distanz des kurzen Referenzarms befindet. Der Strich der Referenzspiegelebene 2 RSE2 zeigt, an welchem Ort des Objektarms sich die optische Distanz des langen Referenzarms befindet. MB1 liegt im vorderen Augensegment. MB1 ist der Messbereich (Scantiefe), der vom kurzen Referenzarm zur Verfügung gestellt wird. In MB1 werden alle Objekte und Augenstrukturen gemessen, die sich von der Vorderfläche der Kornea bis zur Rückfläche der Linse erstrecken, in den meisten Fällen handelt es sich dabei um die Korneavorderfläche, Kornearückfläche, Linsenvorderfläche und Linsenrückfläche. Es kann sich aber auch um einen Flapschnitt innerhalb der Kornea oder um die Sklera oder Iris handeln. MB2 liegt im hinteren Augensegment. MB2 ist der Messbereich (Scantiefe), der vom langen Referenzarm zur Verfügung gestellt wird. In MB2 werden alle Objekte und Augenstrukturen gemessen, die sich hinter der Rückfläche der Linse befinden, in den meisten Fällen handelt es sich dabei um die Retina. Es können im hinteren Messbereich aber auch (krankhafte) Veränderungen der Glaskörperstruktur gemessen werden.

Obige Überlegungen zeigen also, dass die Referenzspiegelebene z.B. nicht innerhalb der Kornea liegen darf, weil sonst nicht mit Sicherheit entschieden werden kann, ob ein Echtsignal der Korneavorderseite oder ein Spiegelsignal der Kornearückseite oder ob ein Echtsignal der Kornearückseite oder ein Spiegelsignal der Korneavorderseite vorliegt. Aus denselben Gründen darf sich die Referenzspiegelebene weder innerhalb des Kammerwassers noch innerhalb der Linse noch innerhalb des Glaskörpers befinden.

Weil die Sensitivität der Messung mit zunehmendem Abstand der Signale von der Referenzspiegelebene abnimmt, und weil die zu messenden Achslängen einen grossen Bereich von typ. 14 - 34 mm umfassen, kann die Sensitivität des Retinasignals stark erhöht werden, wenn der Referenzspiegel in der Position der am längsten zu messenden Augen schrittweise in Richtung der natürlichen Linse so weit verschoben wird, bis das Signal der Retina maximal ist. Deshalb hat der Referenzarm, der für die Messung der Retina zuständig ist, einen Verschiebemechanismus, der den Referenzspiegel in Richtung des einfallenden Referenzstrahls verschiebt.

Es gibt mathematische Algorithmen, die es erlauben, die Spiegelterme um einen Faktor von etwa 1000 abzuschwächen. Dadurch wird es möglich, das Messobjekt in mehr als 2 Messbereiche aufzuteilen, wobei das Risiko von Fehlmessungen infolge der geringen Amplitude der Spiegelterme stark verringert wird. Werden z.B. 4 Messbereiche verwendet, so müssen nacheinander 4 Referenzarme freigeschaltet werden. Das erleichtert die Anforderungen an die Kohärenz der abstimmbaren Lichtquelle. Diese mathematischen Algorithmen sind aber bei schnell bewegten Objekten wie z.B. dem Auge nicht immer praktikabel.

In den nachfolgenden Absätzen sind nun die einzelnen Figuren im Detail beschrieben:

### Ausführungsvariante 1

Eine erste Ausführungsvariante der Erfindung ist in Figur 1 ersichtlich. Lichtstrahlen sind als gestrichelte Linien, elektrische Leitungen als ausgezogene Linien und optische Fasern als dicke ausgezogene Linien dargestellt. Eine abstimmbare Lichtquelle ALQ emittiert eine schmale Spektrallinie (Laserlinie). Über einen 2x2 Faserkoppler FK1, zwei Strahlenteiler ST1 und ST2 wird das Licht in die Referenzarme und in den Objektarm geleitet. Im Objektarm gelangt das Licht der abstimmbaren Lichtquelle über einen Polarisationskontroller PK1, über einen Fokusschalter FS, einen Scanner S, eine Scanoptik SO und einen dritten Strahlenteiler ST3 zum Messobjekt, in diesem Falle zum menschlichen Auge. Strahlenteiler ST3 ist ein Wellenlängen-selektiver Strahlenteiler, der das sichtbare Licht zur Kamera K reflektiert, und infrarotes Licht, das normalerweise für die OCT-Lichtquellen verwendet wird, transmittiert. Der Scanner lenkt den Lichtstrahl in ein oder zwei lateralen Dimensionen über die Kornea, von wo der Lichtstrahl ins Auge gebrochen wird. Bei jeder Brechungsindexänderung im Auge wird ein Teil des Lichtes zurückreflektiert. Das zurückreflektierte Licht geht den gleichen Weg zurück bis zum Strahlenteiler ST2. Eine Kamera K nimmt ein 2-dimensionales Bild des Vorderteils des Auges auf, das dem Benutzer auf einem Monitor M zur Verfügung gestellt wird. Das auf dem Monitor dargestellte Kamerabild erlaubt es dem Benutzer, das Messgerät mit Hilfe des Kreuzschlittens KS so vor dem Auge des Patienten zu positionieren, dass die Messung auf das Auge zentriert ist.

Im Referenzarm gelangt das Licht über einen Scannerspiegel SS, eine fokussierende Optik 03, in ein Glassubstrat DK, dessen Rückfläche mit einem Referenzspiegel RS2 beschichtet ist. Das Glassubstrat DK dient als Dispersionskompensator für die an der Retina des Messobjektes reflektierten Signale. Die Dispersion, die im Auge entsteht, kann durch ein geeignetes im Referenzarm eingefügtes Glassubstrat DK teilweise kompensiert werden, was das Interferenzsignal der Retina erhöht. Das von diesem Referenzspiegel RS1 zurückreflektierte Licht geht den gleichen Weg zurück bis zum Strahlenteiler ST2. Der Pfeil über dem Referenzspiegel RS2 soll andeuten, dass die Ausgangsposition des langen Referenzarms der Referenzspiegelebene RSE2 entspricht, die unmittelbar hinter der längsten zu messenden Achslänge des Auges liegt. Durch das Vorwärtsschieben des Referenzspiegels RS2 wird das Signal der Retina maximiert, wenn der optische Weg im langen Referenzarm, gemessen vom Strahlenteiler ST2 bis zum Referenzspiegel RS2, genau dem optischen Weg im Objektarm, gemessen vom Strahlenteiler ST2 bis zur Retina, entspricht.

In der in Fig. 1 gezeigten Position des Scannerspiegels SS wird das Licht in den langen Referenzarm geleitet, was dazu führt, dass das hintere Segment des Auges (Glaskörper und die Retina) gemessen wird. Der Scannerspiegel SS fährt abwechslungsweise zwei Positionen an. In der einen Position wird der Referenzstrahl senkrecht auf den Referenzspiegel RS2 gelenkt (siehe Fig. 1), in der anderen Position wird er senkrecht auf den Referenzspiegel RS1 gelenkt. Wenn der Referenzstrahl über die Optik 02 auf den Referenzspiegel RS1 fokussiert wird, wird das Auge von der Korneavorderseite bis zur Linsenrückfläche vermessen. Mit Hilfe der Polarisationskontroller PK1, PK2 und PK3 bestehend aus den nacheinander platzierten Komponenten Viertelwellenplatte, Halbwellenplatte und Viertelwellenplatte kann die Amplitude der Interferenzsignale maximiert werden.

Im Strahlenteiler ST2 kommt es zur Interferenz des vom Messobjekt und des vom Referenzspiegel reflektierten Lichtes. Am Strahlenteiler ST2 wird das Licht aufgespaltet in einen Teil, der zur Photodiode 1 PD1 geht und einen anderen Teil, der via Strahlenteiler ST1 zur Photodiode 2 PD2 gelangt. Die Interferenzsignale der Photodiode 1 PD1 und 2 PD2 haben eine Phasendifferenz von 180°. Diese Phasendifferenz in Kombination mit den zwei entgegengesetzt geschalteten Photodioden 1 PD1 und 2 PD2 einer sogenannten balancierten Detektion BD1 ermöglicht es, den Gleichstromanteil der inkohärent überlagerten optischen Signale zu unterdrücken, ohne das Interferenzsignal zu beeinträchtigen.

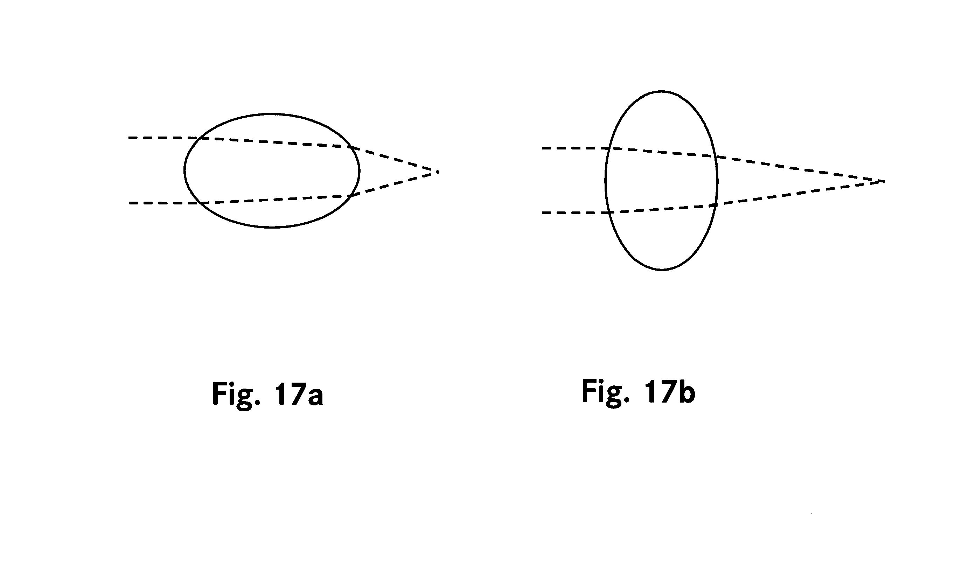

Der Fokusschalter FS schaltet den Fokus des Messstrahls in zwei oder mehrere axiale Positionen im Auge. Vorzugsweise wird je eine Fokusposition im vorderen Messbereich MB1 und hinteren Messbereich MB2 angefahren. Die Umschaltung der Fokusposition muss synchronisiert sein mit der Umschaltung des Scannerspiegels SS. Mögliche Ausführungen eines Fokusschalters sind Flüssiglinsen, die ihre Oberflächenform verändern, oder Flüssigkristalle, die ihren Brechungsindex ändern, oder Linsen, deren Position in Ausbreitungsrichtung des Lichtes z.B. durch einen Piezoaktor verstellt wird, oder optische Komponenten, die in den Strahlengang des Objektstrahls abwechslungsweise ein- und ausgeschwenkt werden. Eine weitere Möglichkeit, die Brennweite zu verändern, ist die Verwendung einer Linse, die, wenn der Lichtstrahl diese in der Position 1 durchläuft, eine Brennweite 1 aufweist (siehe Fig. 17a), und wenn die Linse um z.B. 90° in eine Position 2 gedreht wird, eine von der Brennweite 1 unterschiedliche

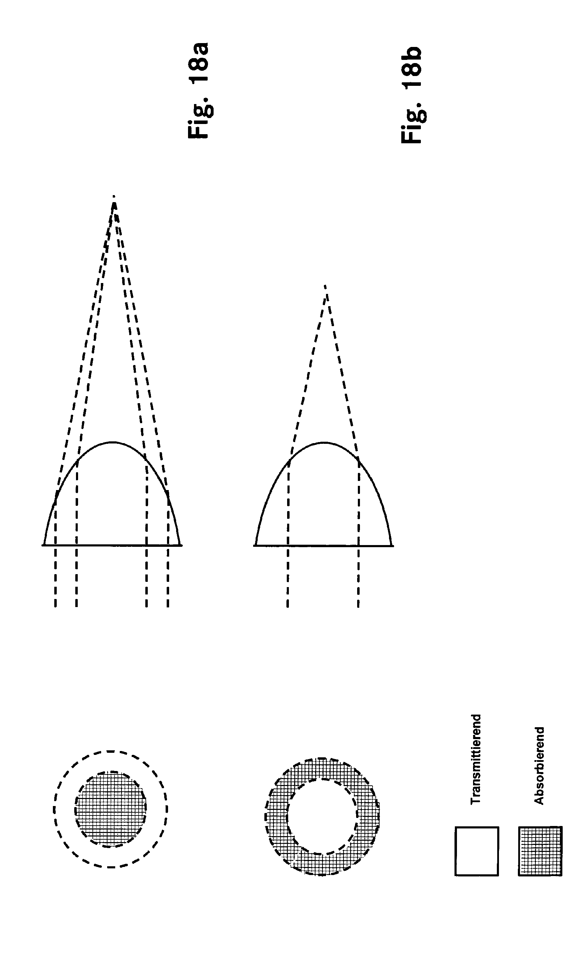

Brennweite 2 für denselben Lichtstrahl aufweist (siehe Fig. 17b). Die Drehachse der Linse ist dabei senkrecht zur Ausbreitungsrichtung des Lichts, das von der Linse fokussiert wird. Als zusätzliche Ausführungsvariante eines Fokusschalters ist ein Strahlmodulator denkbar, der den Strahlquerschnitt eines Lichtstrahls abwechslungsweise auf eine innere zentrale Scheibe oder einen äusseren ringförmigen Bereich einschränkt. Das zentrale oder das ringförmige Strahlbündel trifft auf eine Linse, die in einem zentralen Bereich und in einem äusseren ringförmigen Bereich zwei unterschiedliche Brennweiten besitzt. Dadurch wird das zentrale und das ringförmige Strahlenbündel von zwei unterschiedlichen Linsenbereichen mit unterschiedlicher Brennweite gebrochen. Dadurch entstehen zwei in Ausbreitungsrichtung des Lichtes unterschiedlich positionierte Foki (siehe Fig. 18a, 18b). Der Modulator kann z.B. ein Flüssigkristall sein, der zu einem bestimmten Zeitpunkt den äusseren ringförmigen Bereich Licht-transmittierend und die zentrale Scheibe Licht-absorbierend schaltet, und der zu einem späteren Zeitpunkt den äusseren ringförmigen Bereich Licht-absorbierend und die zentrale Scheibe Licht-transmittierend schaltet.

Die Lichtwellenlänge als Funktion der Zeit kann z.B. in einem Mach-Zehnder Interferometer gemessen und als elektronisches Signal (sogenannte k-Clock) in die Signalverarbeitungsstufe SV eingegeben werden. Das Mach-Zehnder Interferometer besteht aus zwei 2x2 Faserkopplern FK4 und FK5. Die Signale des Mach-Zehnder-Interferometers werden in den entgegengesetzt geschalteten Photodioden PD3 und PD4 und der balancierten Detektion BD2 vom Gleichstromanteil befreit. Der Ausgang der balancierten Detektion BD2 ist die k-Clock. Mit Hilfe des Polarisationskontrollers PK4 wird die Amplitude der auf die zwei Photodioden PD3 und PD4 geleiteten Interferenzsignale maximiert.

Nach der balancierten Detektion BD1 wird das Signal einer Verstärkerstufe VS zugeführt, bevor es in einem analog-digital Wandler AD digitalisiert wird. In der nächsten Stufe, der digitalen Signalverarbeitung SV, werden für jede einzelne Position des Scanners S und des Spiegelscanners SS die zeitlichen Schwebungssignale anhand der gemessenen Lichtwellenlänge als Funktion der Zeit linearisiert und Fourier-transformiert. In weiteren Verarbeitungsschritten können diese einzelnen A-Scans gemittelt, geglättet etc werden. Die A-Scans, die bei jeder Position des Scanners S und des Scannerspiegels SS entstehen, müssen im Raum richtig nebeneinandergelegt werden. So entsteht ein Satz von A-Scans im 2 oder 3-dimensionalen Raum, je nach dem ob der Scanner S eine oder zwei transversale Richtungen abscannt.

In der nächsten Stufe der 3D-Auswertung 3D werden in diesem Satz von A-Scans die Oberflächen der Kornea, der Linse und der Retina berechnet (Segmentation). Danach werden die der Vorderfläche der Kornea nachfolgenden Flächen (Rückseite der Kornea, Vorderfläche der Linse, Rückfläche der Linse und Retina) berechnet, indem die Richtungen der A-Scans anhand der Oberflächenkrümmungen und Brechungsindices der vorgelagerten Flächen neu berechnet. Bei dieser Neuberechnung wird die Brechung der Lichtstrahlen an den einzelnen Oberflächen berücksichtigt (sogenannte Refraktionskorrektur). Ausserdem kann die Beugung der Lichtstrahlen an der Pupille mitberücksichtigt werden, was vor allem bei Pupillen von weniger als 3 mm Durchmesser sinnvoll ist. Die so erhaltenen Flächen können nun noch weiter verarbeitet werden, z.B. indem sie nach einem Satz von orthonormalen Funktionen entwickelt werden (z.B. Zernike-Polynome).

Im Berechnungsblock IOL werden die im 3-dimensionalen Raum aufgespannten Flächen der Korneavorderseite, Kornearückseite, Linsenvorderseite und Linsenrückseite und Retina von virtuellen Lichtstrahlen bestrahlt, die dem Brechungsgesetz und Beugungsgesetz folgen. Dieses sogenannte Ray-Tracing an den virtuellen Oberflächen des menschlichen Auges erlaubt nun die Berechnung einer intraokularen Linse, einer photorefraktiven Korrektur der Kornea etc, indem die örtliche Ausdehnung des auf die Retina abgebildeten Strahlenmusters minimiert bzw. optimiert wird.

Die 3D-Auswertung 3D und der Berechnungsblock IOL wird normalerweise auf einem Personal Computer PC durchgeführt.

### Ausführungsvariante 2

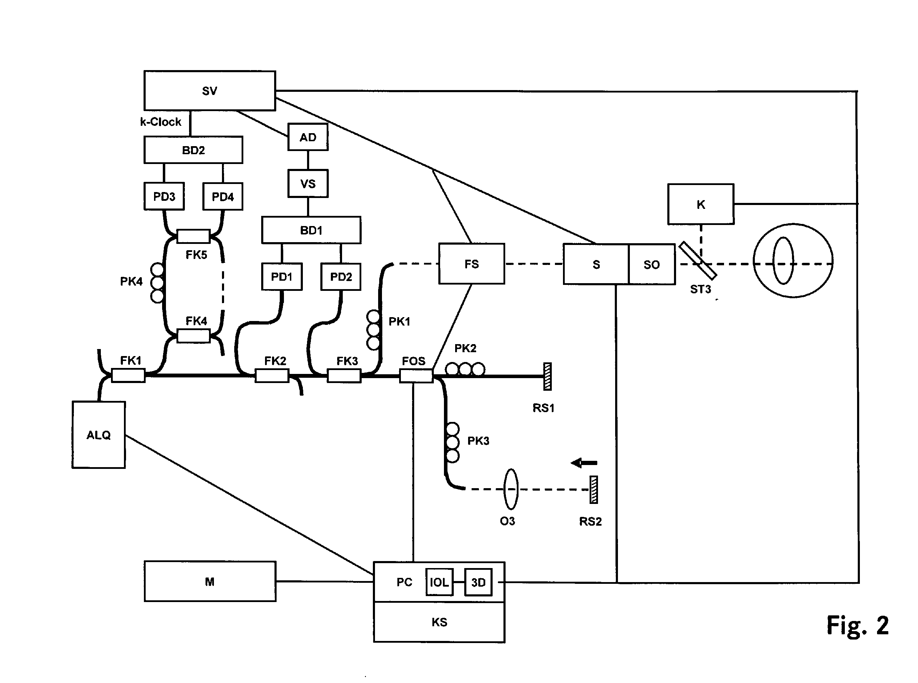

Eine zweite Ausführungsvariante der Erfindung ist in Figur 2 gezeichnet. Eine abstimmbare Lichtquelle ALQ emittiert eine schmale Spektrallinie (Laserlinie). Über drei 2x2 Faserkoppler FK1, FK2 und FK3 wird das Licht zu den Referenzarmen und zum Objektarm geleitet. Im Objektarm gelangt das Licht der abstimmbaren Lichtquelle ALQ über einen faseroptischen Polarisationskontroller PK1, über einen Fokusschalter FS, einen Scanner S, eine Scanoptik SO und einen Strahlenteiler ST3 zum Messobjekt, in diesem Falle zum menschlichen Auge. Der Scanner S lenkt den Lichtstrahl in ein oder zwei lateralen Dimensionen über die Kornea, von wo der Lichtstrahl ins Auge gebrochen wird. An jeder Brechungsindexänderung im Auge wird ein Teil des Lichtes zurückreflektiert. Das zurückreflektierte Licht geht den gleichen Weg zurück bis zum Faserkoppler FK3. Eine Kamera K nimmt ein 2-dimensionales Bild des Vorderteils des Auges auf, das dem Benutzer auf einem Monitor M zur Verfügung gestellt wird. Das auf dem Monitor dargestellte Kamerabild erlaubt es dem Benutzer, das Messgerät mit Hilfe des Kreuzschlittens KS so vor dem Auge des Patienten zu positionieren, dass die Messung auf das Auge zentriert ist.

Ein Ausgang des Faserkopplers FK3 führt zu einem faseroptischen Schalter FOS (englisch: fiberoptic switch), der das Licht abwechslungsweise in einen langen und einen kurzen Referenzarm leitet. Der kurze Referenzarm bestehend aus dem faseroptischen Polarisationskontroller PK2 und einem direkt auf die Faserendfläche aufgetragenen Referenzspiegel RS1 erlaubt die Interferenz der im kurzen Referenzarm reflektierten Strahlung mit der im vorderen Augensegment reflektierten Strahlung. Der lange Referenzarm bestehend aus dem faseroptischen Polarisationskontroller PK3, einer Optik 03 und einem Referenzspiegel RS2 erlaubt die Interferenz der im langen Referenzarm reflektierten Strahlung mit der im hinteren Augensegment reflektierten Strahlung. Die Optik 03 fokussiert den Referenzstrahl auf den Referenzspiegel RS2. Die übrigen Komponenten in Fig. 2 sind identisch mit denjenigen in Fig. 1 und sind in der Beschreibung der Fig. 1 erklärt.

### Ausführungsvariante 3

In Fig. 3 Ist eine Ausführungsvariante eines mechanisch synchronisierten Fokus- und Distanzschalters gezeigt. Eine optische Komponente bestehend aus einer Art Prisma P ist mit einer Glasplatte GP verbunden. Durch das Ein- und Ausschwenken dieser Komponente in den Strahlengang des Objekt- und Referenzarms wird synchron der Fokus und der Messbereich zwischen dem vorderen Augensegment und dem hinteren Augensegment hin-und hergeschaltet. Der Referenzspiegel RS1 wird an die Position des höchsten Retinasignals bewegt, wenn das Prisma P in den Referenzstrahl eingeführt ist. In diesem Fall ist der Referenzarm lang und das hintere Augensegment wird gemessen. Befindet sich das Prisma P nicht im Strahlengang des Referenzarms, so wird das vordere Augensegment gemessen. In dieser Position ist es normalerweise nicht nötig, den Referenzspiegel zu bewegen, um die Signale des vorderen Augensegments zu optimieren. Die übrigen Bauteile dieser Ausführungsvariante sind in Fig. 2 beschrieben.

### Ausführungsvariante 4

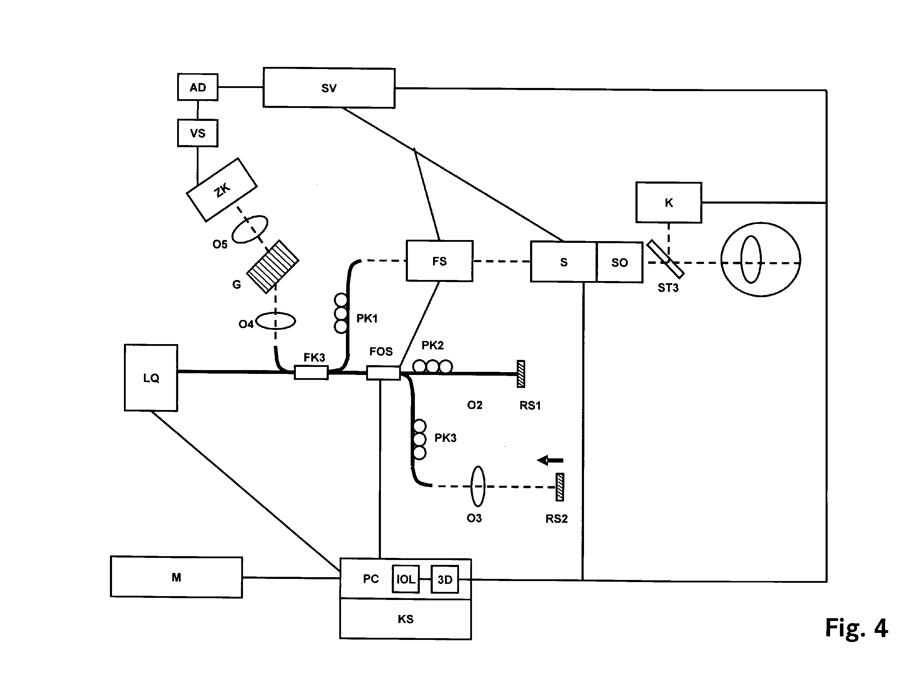

Eine weitere, vierte Ausführungsvariante der Erfindung ist in Figur 4 dargestellt. In Fig. 4 ist ein Messgerät basierend auf spektraler OCT gezeigt. Im Gegensatz zu dem in Fig. 2 dargestellte Aufbau ist die Lichtquelle LQ nicht abstimmbar. Die Lichtquelle LOG emittiert ein zeitlich konstantes Spektrum. Weil das Spektrum nicht ändert, braucht diese Ausführung keine k-Clock, insbesondere sind also das in Fig. 2 vorhandene Mach-Zehnder-Interferometer, der Faserkoppler FK1 und die balancierte Detektion BD2 samt dem Faserkoppler FK2 nicht nötig und sie sind deshalb in Fig. 4 nicht vorhanden. Abgesehen vom Quellenarm besteht der einzige Unterschied zu der in Fig. 2 gezeigten Ausführung in der Detektion der Interferenzsignale. Die Lichtinterferenz wird im Detektorarm über eine Optik 04 auf ein Gitter G gebracht. Das Gitter lenkt die verschiedenen Wellenlängen des Spektrums in verschiedene Richtungen ab. Das bedeutet, dass jedes Pixel der Zeilenkamera ZK einen bestimmten Ausschnitt aus dem Wellenlängenspektrum der Lichtquelle LQ detektiert. Die Optik 05 fokussiert die verschiedenen Wellenlängen des Spektrums der Lichtquelle LQ auf die Zeilenkamera ZK. Alle übrigen Komponenten der Fig. 4 sind identisch mit denjenigen in Fig. 2 und sind in der Beschreibung der Fig. 2 erklärt.

### Ausführungsvariante 5

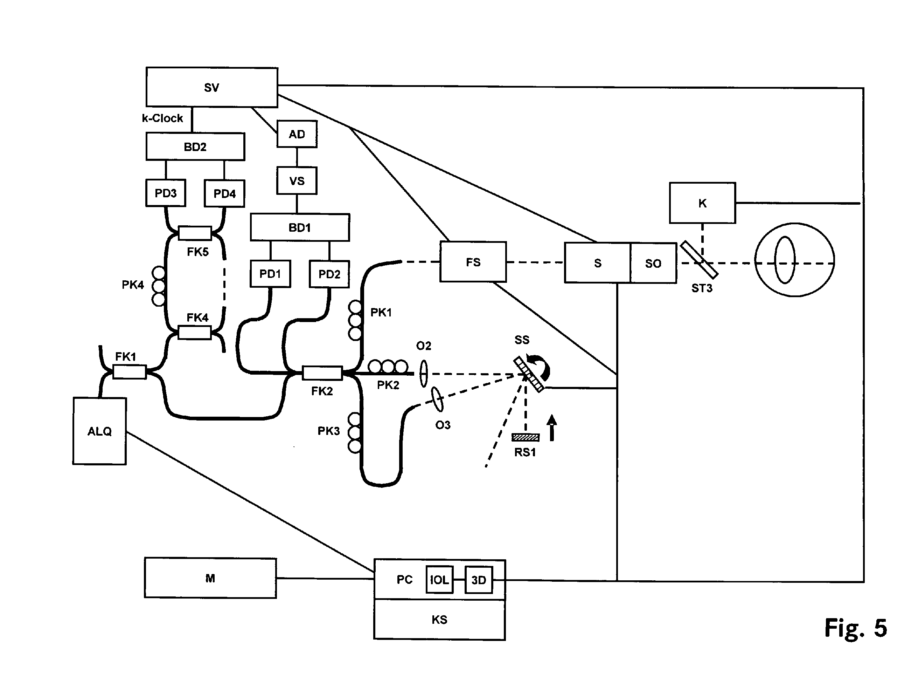

In Fig. 5 ist ein OCT mit abstimmbarer Lichtquelle ALQ gezeigt. Anstelle der zwei 2x2 Faserkoppler FK2 und FK3, wie sie in Fig. 2 dargestellt ist, wird nur ein 3x3 Faserkoppler FK2 verwendet. Der 3x3 Faserkoppler FK2 teilt das Licht in einen Objektarm und zwei Referenzarme auf. Der Spiegelscanner lenkt abwechslungsweise das Licht des kurzen Referenzarms und dasjenige des langen Referenzarms auf den Referenzspiegel RS1. Die Polarisationskontroller PK1, PK2 und PK3 werden verwendet, um die Polarisation des kurzen Referenzarms bzw. des langen Referenzarms an die Polarisation des Objektarms anzugleichen. Die Optik 02 fokussiert das Licht des kurzen Referenzarms auf den Referenzspiegel RS1. Die Optik 03 fokussiert das Licht des langen Referenzarms auf den Referenzspiegel RS1. Die übrigen Komponenten sind schon in den Beschreibung der Fig. 2 und Fig. 1 erklärt.

### Ausführungsvariante 6

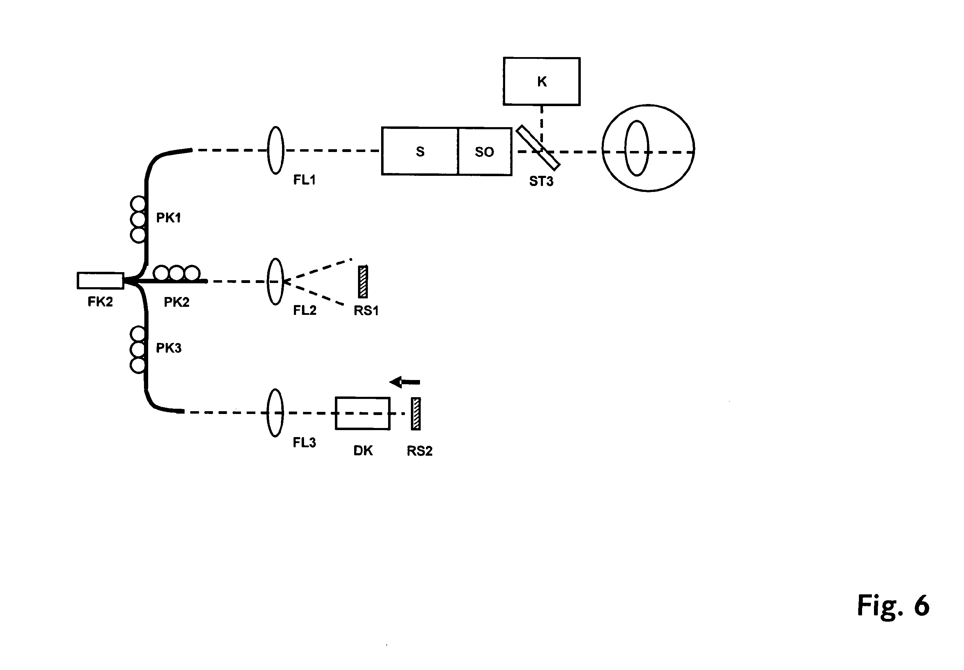

Aus Gründen der Einfachheit zeigt Fig. 6 nur die zwei Referenzarme und den Objektarm des OCTs. Die übrigen Komponenten sind weggelassen. Der in Fig. 6 weggelassene Quellenarm kann z.B. identisch mit dem in Fig. 5 dargestellten Quellenarm sein. Der in Fig. 6 weggelassene Detektionsarm kann z.B. identisch mit dem in Fig. 5 dargestellten Detektionsarm sein. Fig. 6 zeigt drei Flüssiglinsen FL1, FL2 und FL3, die periodisch und synchronisiert ihre Brechkraft ändern. FL2 fokussiert und defokussiert den Strahl des kurzen Referenzarms auf den Referenzspiegel RS1 mit einer bestimmten Rate, FL3 fokussiert und defokussiert den Strahl des langen Referenzarms auf den Referenzspiegel RS2 mit derselben Rate, dessen Phase aber um 180° verschoben ist. Dadurch ist immer nur einer der beiden Referenzarme interferenzfähig mit dem aus dem Objektarm reflektierten Licht. In Fig. 6 ist gezeigt, wie die Flüssiglinse FL3 den Strahl des langen Referenzarms, der den Dispersionskompensator DK enthält, auf den Referenzspiegel RS1 fokussiert. Dadurch wird ein sehr grosser Anteil der Lichtleistung des langen Referenzarms zurück in die Faser des Faserkopplers FK2 eingekoppelt. Somit ist in der Momentaufnahme, die die Fig. 6 zeigt, der lange Referenzarm interferenzfähig. Das heisst Signale vom hinteren Augensegment werden gemessen. Man kann deshalb sagen, dass in der gezeigten Momentaufnahme der lange Referenzarm geöffnet ist.

In Fig. 6 ist gezeigt, wie die Flüssiglinse FL2 den Referenzstrahl defokussiert. Dadurch gelangt kein oder nur ein verschwindend kleiner Anteil der Referenzstrahllichtleistung zurück in die Faser des Faserkopplers FK2. Somit ist der kurze Referenzarm nicht interferenzfähig mit dem aus dem Objektarm reflektierten Licht, das heisst, dass in diesem Moment keine Signale aus dem vorderen Augensegment gemessen werden können. Man kann deshalb sagen, dass der kurze Referenzarm geschlossen ist.

Die Polarisationskontroller PK1, PK2 und PK3 werden verwendet, um die Polarisation des kurzen Referenzarms bzw. des langen Referenzarms an die Polarisation des Objektarms anzugleichen.

Die Flüssiglinse FL1 fokussiert die Messstrahlung abwechslungsweise in das vordere und hintere Augensegment. Die drei Flüssiglinsen FL1, FL2 und FL3 arbeiten synchron, das heisst, fokussiert FL1 den Strahl ins vordere Augensegment, so fokussiert FL2 den Referenzstrahl auf den Referenzspiegel RS1 und FL3 defokussiert den Referenzstrahl auf dem Referenzspiegel RS2. Fokussiert FL1 den Strahl ins hintere Augensegment, so defokussiert FL2 den Referenzstrahl auf dem Referenzspiegel RS1 und FL3 fokussiert den Referenzstrahl auf den Referenzspiegel RS2.

### Ausführungsvariante 7

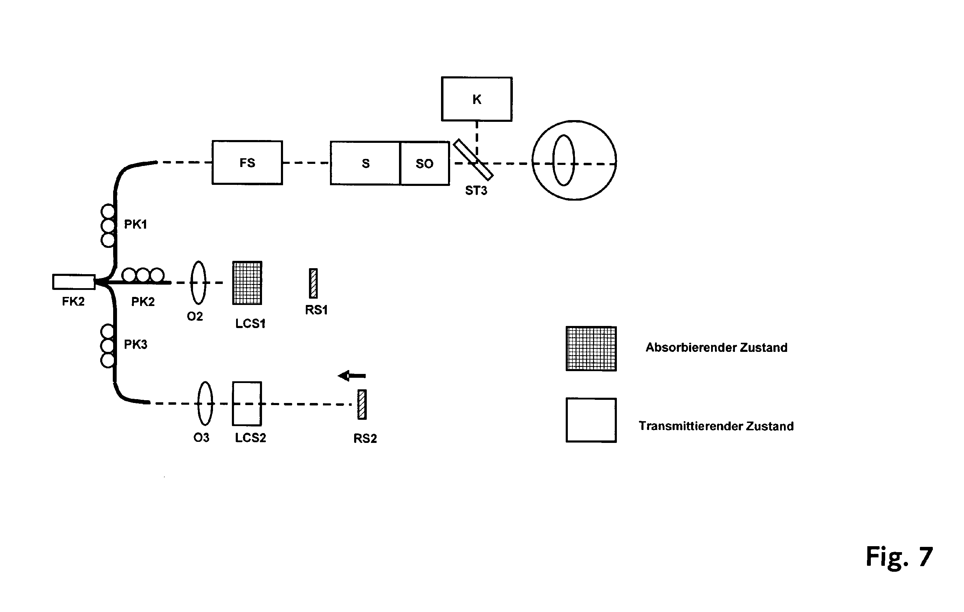

Aus Gründen der Einfachheit zeigt Fig. 7 nur die zwei Referenzarme und den Objektarm des OCTs. Die übrigen Komponenten sind weggelassen. Der in Fig. 7 weggelassene Quellenarm kann z.B. identisch mit dem in Fig. 5 dargestellten Quellenarm sein. Der in Fig. 7 weggelassene Detektionsarm kann z.B. identisch mit dem in Fig. 5 dargestellten Detektionsarm sein. Das Öffnen und Schliessen der zwei Referenzarme wird mit zwei Flüssigkristallen LCS1 und LCS2 bewerkstelligt. LCS1 öffnet und schliesst den kurzen Referenzarm, LCS2 öffnet und schliesst den langen Referenzarm. Zu jeder Zeit ist nur einer der zwei Flüssigkristalle transmittierend, so dass immer nur einer der zwei Referenzarme mit der aus dem Objektarm reflektierten Strahlung interferenzfähig ist. In Fig. 7 ist kein Dispersionskompensator eingezeichnet. Bis zu einem gewissen Grad kann auch die optische Faser des langen Referenzarms als Dispersionskompensator verwendet werden, indem der Weg im hinteren Augensegment durch einen entsprechenden Weg in der Faser kompensiert wird.

Die Polarisationskontroller PK1, PK2 und PK3 werden verwendet, um die Polarisation des kurzen Referenzarms bzw. des langen Referenzarms an die Polarisation des Objektarms anzugleichen. Die Optik 02 fokussiert das Licht des kurzen Referenzarms auf den Referenzspiegel RS1. Die Optik 03 fokussiert das Licht des langen Referenzarms auf den Referenzspiegel RS2.

### Ausführungsvariante 8

Ausführungsvariante 8 zeigt einen Aufbau mit einem Referenzarm und zwei Objektarmen. Aus Gründen der Einfachheit zeigen Fig. 8a und 8b den Quellenarm und den Detektionsarm nicht. Der in Fig. 8a und 8b weggelassene Quellenarm kann z.B. identisch mit dem in Fig. 5 dargestellten Quellenarm sein. Der in Fig. 8a und 8b weggelassene Detektionsarm kann z.B. identisch mit dem in Fig. 5 dargestellten Detektionsarm sein. Ein Schalter öffnet und schliesst abwechslungsweise eine der zwei Objektarme. Zu jeder Zeit ist nur einer der zwei Objektarme geöffnet, so dass immer nur einer der zwei Objektarme mit der aus dem Referenzarm reflektierten Strahlung interferenzfähig ist. Wenn der obere der zwei Objektarme geöffnet (so wie in Fig. 8a dargestellt), so ist das im vorderen Augensegment reflektierte Licht interferenzfähig mit dem vom Referenzarm reflektierten Licht. Die Differenz der optischen Weglänge zwischen dem Weg von FK2 über S1 bis zum Schalter (langer Objektarm) und dem Weg von FK2 über 02 und dem Schalter (kurzer Objektarm) entspricht der optischen Länge des vorderen Augensegments. In Fig. 8a ist gezeigt, dass das Licht des kurzen Objektarms beim Schalter absorbiert wird. Der Schalter kann z.B. ein Spiegel sein, der abwechslungsweise zwei verschiedene Winkelpositionen anfährt. Klappt der Spiegel nach unten, so wird der kurze Objektarm geöffnet, siehe Fig. 8b. In dieser Position des Spiegels wird das Licht des langen Objektarms im Absorber A 1 absorbiert.

Der Referenzspiegel RS1 wird nur verschoben, wenn der kurze Objektarm geöffnet ist. Die Verschiebung des Referenzspiegels beginnt bei einer Position, die für die Messung der längsten zu messenden Augen verwendet wird. Durch das Verschieben des Referenzspiegels in Richtung der Optik 03 wird diejenige Position des Referenzspiegels angefahren, bei der das Retinasignal maximal ist. Das Maximieren des Retinasignals ist in denjenigen Augen nötig, bei denen durch das Vorhandensein von Katarakt das Retinasignal stark abgeschwächt wird.

Die Polarisationskontroller PK1, PK2 und PK3 werden verwendet, um die Polarisation des kurzen Objektarms bzw. des langen Objektarms an die Polarisation des Referenzarms anzugleichen. Die Optik 01 fokussiert das Licht des langen Objektarms ins vordere Augensegment. Die Optik 02 fokussiert das Licht des kurzen Objektarms ins hintere Augensegment. Die Optik 03 fokussiert das Licht des Referenzarms auf den Referenzspiegel RS1.

### Ausführungsvariante 9

Ausführungsvariante 9 zeigt einen Aufbau mit einem Referenzarm und zwei Objektarmen. Aus Gründen der Einfachheit zeigen Fig. 9a und 9b den Quellenarm und den Detektionsarm nicht. Der in Fig. 9a und 9b weggelassene Quellenarm kann z.B. identisch mit dem in Fig. 5 dargestellten Quellenarm sein. Der in Fig. 9a und 9b weggelassene Detektionsarm kann z.B. identisch mit dem in Fig. 5 dargestellten Detektionsarm sein. Zwei Schalter in Form von zwei Flüssigkristallen LCS1 und LCS2 öffnen und schliessen abwechslungsweise einen der zwei Objektarme. Zu jeder Zeit ist nur einer der zwei Objektarme geöffnet, so dass immer nur einer der zwei Objektarme mit der aus dem Referenzarm reflektierten Strahlung interferenzfähig ist. Wenn der obere der zwei Objektarme geöffnet (so wie in Fig. 9a dargestellt), so ist das im vorderen Augensegment reflektierte Licht interferenzfähig mit dem vom Referenzarm reflektierten Licht. Die Differenz der optischen Weglänge zwischen dem Weg von FK2 über S1 bis zum Strahlenteiler ST4 (langer Objektarm) und dem Weg von FK2 über 02 und dem Strahlenteiler ST4 (kurzer Objektarm) entspricht der optischen Länge des vorderen Augensegments. In Fig. 9a ist gezeigt, dass das Licht des kurzen Objektarms im Flüssigkristall LCS2 absorbiert wird. Fig. 9b zeigt eine Momentaufnahme, in der das Licht des kurzen Objektarms auf das Auge geleitet wird. In dieser Einstellung ist das Licht aus dem kurzen Referenzarm interferenzfähig mit dem aus dem Objektarm reflektierten Licht.

In Fig. 9b ist gezeigt, dass das Licht des langen Objektarms im Flüssigkristall LCS1 absorbiert wird.

Der Referenzspiegel RS1 wird nur verschoben, wenn der kurze Objektarm geöffnet ist. Die Verschiebung des Referenzspiegels beginnt bei einer Position, die für die Messung der längsten zu messenden Augen verwendet wird. Durch das Verschieben des Referenzspiegels in Richtung der Optik 03 wird diejenige Position des Referenzspiegels angefahren, bei der das Retinasignal maximal ist. Das Maximieren des Retinasignals ist in denjenigen Augen nötig, bei denen durch das Vorhandensein von Katarakt das Retinasignal stark abgeschwächt wird.

Die Polarisationskontroller PK1, PK2 und PK3 werden verwendet, um die Polarisation des kurzen Objektarms bzw. des langen Objektarms an die Polarisation des Referenzarms anzugleichen. Die Optik O1 fokussiert das Licht des langen Objektarms ins vordere Augensegment. Die Optik 02 fokussiert das Licht des kurzen Objektarms ins hintere Augensegment. Die Optik 03 fokussiert das Licht des Referenzarms auf den Referenzspiegel RS1.

### Ausführungsvariante 10

Fig. 10 zeigt einen Aufbau mit zwei polarisierenden Strahlenteilerwürfeln PST1 und PST2 und zwei Flüssigkristallen LCS1 und LCS2. In Fig. 10 sind zwei Objektarme und ein Referenzarm vorhanden. Von der Lichtquelle ALQ bis zum Strahlenteiler ST2 entspricht die in Fig. 10 gezeigte Ausführungsvariante der Ausführungsvariante, die in Fig. 1 gezeigt ist. Die Optik 02 fokussiert den Referenzstrahl auf den Referenzspiegel RS1. Es sind zwei Objektarme vorhanden, wobei einer der zwei Objektarme eine Umwegeinheit enthält. Die Umwegeinheit besteht aus einem Polarisationskontroller PK2, zwei Umlenkspiegeln S1 und S2, einem Flüssigkristall LCS2 und einer Optik 03. Die Optik 03 fokussiert den Messstrahl ins vordere Augensegment. Die Polarisationskontroller PK1, PK2 und PK3 werden verwendet, um die Polarisation des Referenzarms an die Polarisation des Objektarms mit Umwegeinheit bzw. des Objektarms ohne Umwegeinheit anzugleichen. Der polarisierende Strahlenteilerwürfel PST1 spaltet den einfallenden Messstrahl in einen Strahl mit senkrecht zueinander stehenden Polarisationen auf. Das heisst, dass die

Strahlung, die durch die Umwegeinheit geht senkrecht zur Strahlung polarisiert ist, die nicht durch die Umwegeinheit geht. Der polarisierende Strahlenteilerwürfel PST2 kombiniert die zwei senkrecht zueinander stehenden Polarisationen wieder. Weil das Auge nur relativ schwach doppelbrechend ist, werden die zwei von den Augenstrukturen reflektierten Polarisationen denjenigen Weg durchlaufen, den sie im Hinweg durchlaufen haben. Das heisst: geht die einfallende p-Polarisation durch die Umwegeinheit, so geht auch die Reflexion der auf das Auge einfallenden p-Polarisation durch die Umwegeinheit; und geht die einfallende s-Polarisation nicht durch die Umwegeinheit, so geht auch die Reflexion der auf das Auge einfallenden s-Polarisation nicht durch die Umwegeinheit. Das Licht, das durch die Umwegeinheit geht, misst das vordere Augensegment. Das Licht, das nicht durch die Umwegeinheit geht, misst das hintere Augensegment. In der in Fig. 10 gezeigten Momentaufnahme öffnet der Flüssigkristall LCS1 den Objektarm ohne Umwegeinheit und der Flüssigkristall LCS2 schliesst den Objektarm mit der Umwegeinheit.

### Ausführungsvariante 11

In Fig. 11a und 11b wird ein Aufbau gezeigt, bei dem ein drehbarer Spiegel S1 sowohl als Distanz- als auch als Fokusschalter wirkt. Dies wird ermöglicht, indem der drehbare Spiegel S1 sowohl beide Referenzstrahlen als auch den Objektstrahl beeinflusst.

Aus Gründen der Einfachheit zeigt Fig. 11 a und 11b den Quellenarm und den Detektionsarm nicht. Der in Fig. 11a und 11b weggelassene Quellenarm kann z.B. identisch mit dem in Fig. 5 dargestellten Quellenarm sein. Der in Fig. 11a und 11b weggelassene Detektionsarm kann z.B. identisch mit dem in Fig. 5 dargestellten Detektionsarm sein.

Die Optik O1 lenkt den Objektstrahl auf den Spiegel S1. Die Optik 02 fokussiert den Referenzstrahl des kurzen Referenzarms auf den Referenzspiegel RS1, siehe Fig. 11a. Die Optik 03 fokussiert den Referenzstrahl des langen Referenzarms auf den Referenzspiegel RS1, siehe Fig. 11b. Die Polarisationskontroller PK1, PK2 und PK3 werden verwendet, um die Polarisation der Referenzarme an die Polarisation des Objektarms anzugleichen.

In der Position 1 des Spiegels S1 wird das Licht im kurzen Referenzarm am Referenzspiegel RS1 zurückreflektiert, so dass eine Interferenz zwischen dem aus dem kurzen Referenzarm reflektierten Licht und dem aus dem vorderen Messbereich des Objektarms reflektierten Licht ermöglicht wird. In der Position 1 wird das Licht im langen Referenzarm nicht reflektiert, so dass eine Interferenz zwischen dem aus dem langen Referenzarm reflektierten Licht und dem aus dem hinteren Messbereich des Objektarms reflektierten Licht nicht möglich ist. Vorzugsweise wird das Licht des langen Referenzarms an einem Absorber A1 absorbiert, damit kein Licht aus dem langen Referenzarm in den Faserkoppler FK2 zurückgekoppelt wird. In der Position 1 des Spiegels S1 wird das Licht des Objektarms zur Optik 04 geleitet, die in Kombination mit der Scanoptik SO das Licht im vorderen Messbereich MB1 fokussiert. Der Messstrahl wird vom festen Spiegel S2 auf den Spiegel S3, der sich in Position 1 befindet, gelenkt.

In der Position 2 des Spiegels S1 wird das Licht im langen Referenzarm am Referenzspiegel RS1 zurückreflektiert, so dass eine Interferenz zwischen dem aus dem langen Referenzarm reflektierten Licht und dem aus dem hinteren Messbereich des Objektarms reflektierten Licht ermöglicht wird. In der Position 2 wird das Licht im kurzen Referenzarm nicht reflektiert, so dass eine Interferenz zwischen dem aus dem kurzen Referenzarm reflektierten Licht und dem aus dem vorderen Messbereich des Objektarms reflektierten Licht nicht möglich ist. Vorzugsweise wird das Licht des kurzen Referenzarms an einem Absorber A2 absorbiert, damit kein Licht aus dem kurzen Referenzarm in den Faserkoppler FK2 zurückgekoppelt wird. In der Position 2 des Spiegels S1 wird das Licht des Objektarms direkt zum Spiegel S3 geleitet, der sich nun in der Position 2 befindet. Die Position 2 des Spiegels S3 lenkt den Messstrahl so ab, dass die Ausbreitungsrichtung des Messstrahls nach der Reflexion am Spiegel S3 genau derjenigen Ausbreitungsrichtung entspricht, wenn der Spiegel S1 und der Spiegel S3 sich in Position 1 befinden. In der Position 2 des Spiegels S1 durchläuft der Messstrahl die Optik 04 nicht und wird deshalb im hinteren Messbereich MB2 fokussiert.

### Ausführungsvariante 12

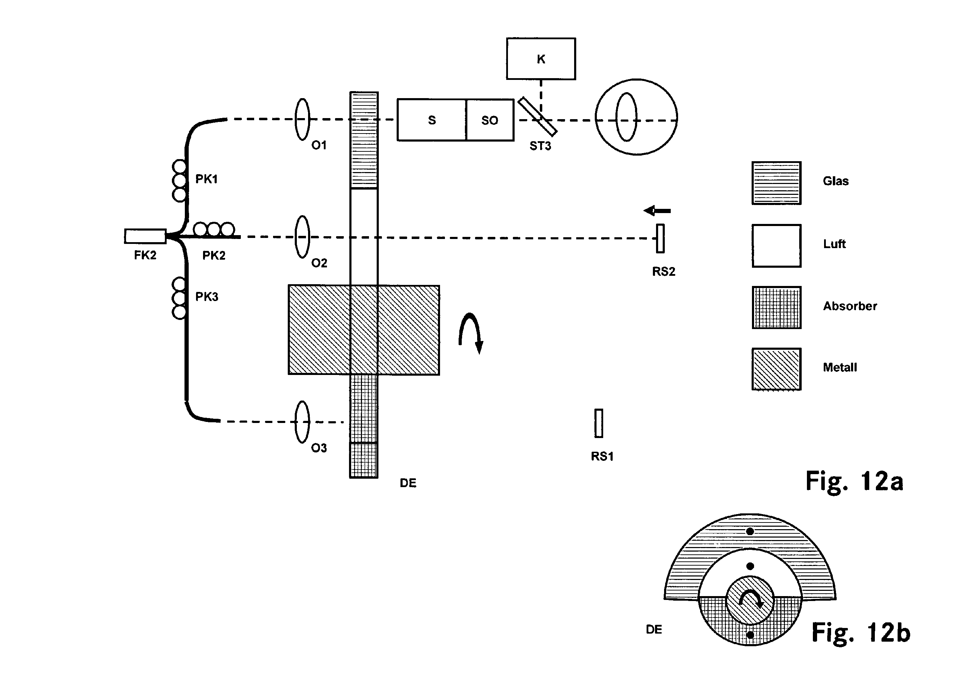

Ausführungsvariante 12 zeigt einen Aufbau mit zwei Referenzarmen und einem Objektarm. Die in Fig. 12a und 12b gezeigte Ausführungsvariante stellen einen mechanisch synchronisierten Fokus- und Distanzschalter dar.

Aus Gründen der Einfachheit zeigt Fig. 12a den Quellenarm und den Detektionsarm nicht. Der in Fig. 12a weggelassene Quellenarm kann z.B. identisch mit dem in Fig. 5 dargestellten Quellenarm sein. Der in Fig. 12a weggelassene Detektionsarm kann z.B. identisch mit dem in Fig. 5 dargestellten Detektionsarm sein. Fig. 12a zeigt ein drehbares Element DE, das aus einer Drehachse, einer halbkreisförmigen Glasplatte, einem halbkreisförmigen Absorber und einem halbkreisförmigen Loch besteht. Das drehbare Element DE schaltet abwechslungsweise während je einer halben Umdrehung einen kurzen und einen langen Referenzarm frei, indem der jeweilige Referenzstrahl entweder vom absorbierenden Material des drehbaren Elements DE absorbiert oder durch das Loch des drehbaren Elements DE transmittiert wird. Zudem fügt das drehbare Element DE während einer halben Umdrehung eine Glasplatte in den Strahlengang des Objektarms ein. Die Dicke der Glasplatte ist so gewählt, dass der Fokus des Messstrahls im hinteren Augensegment zu liegen kommt, wenn der Messstrahl durch die Glasplatte geht (siehe Fig. 12a). In dieser Position des drehbaren Elements DE ist der lange Referenzarm geöffnet und der Strahl des kurzen Referenzarms wird absorbiert. In dieser Position wird das hintere Augensegment gemessen.

Ist das drehbare Element in der Position, in der die Glasplatte sich nicht im Strahlengang des Messstrahls befindet, so liegt der Fokus des Messstrahls im vorderen Augensegment. Der Fokus des Messstrahls springt genau in demjenigen Moment vom vorderen in das hintere Augensegment, wenn der kurze Referenzarm, der für die Messung des vorderen Augensegments verwendet wird, durch den Absorber geschlossen wird und wenn der lange Referenzarm, der für die Messung des hinteren Augensegments verwendet wird, geöffnet wird, indem er sich ungehindert bis zum Referenzspiegel RS2 fortpflanzen kann.

Fig. 12b zeigt das drehbare Element DE aus einer Perspektive, die um 90° gedreht ist verglichen mit der in Fig. 12a gezeigten Perspektive. Die drei schwarzen Punkte zeigen die Durchstosspunkte des Objektarmstrahls und der zwei Referenzarmstrahlen.

### Ausführungsvariante 13

Ausführungsvariante 13 zeigt einen Aufbau mit einem Referenzarm und einem Objektarm.

Aus Gründen der Einfachheit zeigt Fig. 13a den Quellenarm und den Detektionsarm nicht. Der in Fig. 13a weggelassene Quellenarm kann z.B. identisch mit dem in Fig. 3 dargestellten Quellenarm sein. Der in Fig. 12a weggelassene Detektionsarm kann z.B. identisch mit dem in Fig. 3 dargestellten Detektionsarm sein. Fig. 13a zeigt ein drehbares Element DE, das aus einer Drehachse, einer halbkreisförmigen Glasplatte, einem halbkreisförmigen Spiegel und einem halbkreisförmigen Loch besteht. Das drehbare Element DE schaltet abwechslungsweise während je einer halben Umdrehung einen kurzen und einen langen Referenzarm frei, indem der jeweilige Referenzstrahl entweder vom Spiegel des drehbaren Elements DE reflektiert oder durch das Loch des drehbaren Elements DE transmittiert wird. Befindet sich der Spiegel des drehbaren Elements DE im Strahlengang des Referenzarms, so wirkt der Spiegel des drehbaren Elements DE als Referenzspiegel des kurzen Referenzarms.

Zudem fügt das drehbare Element DE während einer halben Umdrehung eine Glasplatte in den Strahlengang des Objektarms ein. Die Dicke der Glasplatte ist so gewählt, dass der Fokus des Messstrahls im hinteren Augensegment zu liegen kommt, wenn der Messstrahl durch die Glasplatte geht. Ist das drehbare Element in der Position, in der die Glasplatte sich nicht im Strahlengang des Messstrahls befindet, so liegt der Fokus des Messstrahls im vorderen Augensegment. Der Fokus des Messstrahls springt also genau in demjenigen Moment vom vorderen in das hintere Augensegment, wenn der Spiegel des drehbaren Elements DE aus dem Referenzarm herausgedreht wird, so dass der lange Referenzarm, der für die Messung des hinteren Augensegments verwendet wird, geöffnet wird, indem sich der Referenzstrahl ungehindert bis zum Referenzspiegel RS2 fortpflanzen kann.

Fig. 13b zeigt das drehbare Element DE aus einer Perspektive, die um 90° gedreht ist verglichen mit der in Fig. 13a gezeigten Perspektive. Die zwei schwarzen Punkte zeigen die Durchstosspunkte des Objektarms und des Referenzarms.

### Ausführungsvariante 14

Ausführungsvariante 14 ist identisch mit der Ausführungsvariante 1 abgesehen von dem einen Unterschied, dass unmittelbar vor dem Auge des Patienten ein Konus oder eine Halbkugel angebracht ist, der ein innenseitiges Muster von konzentrischen dunklen und hellen Ringen aufweist. Dieses Ringmustersystem RMS wird vom Tränenfilm des untersuchten Auges gespiegelt. Das Spiegelbild dieses Ringmustersystems wird von der Kamera K aufgenommen. Aus der Verformung des auf die Kamera abgebildeten Ringmustersystems kann eine Software die Oberflächenform des Tränenfilms bzw. der Korneavorderfläche berechnen. Die mit dem Ringmustersystem gemessene Oberflächenform wird verwendet, um die Messgenauigkeit der OCT-Messung zu verbessern.

### Ausführungsvariante 15

Ausführungsvariante 15 ist in Figur 15 dargestellt. In dieser Ausführung wird die Messung des vorderen Augensegments und des hinteren Augensegments mit einer unterschiedlichen Wellenlänge durchgeführt. Die Verwendung von zwei Wellenlängen kann vorteilhaft sein, weil die maximal zulässige optische Leistung, mit der lebende menschliche Augen gemessen werden können, mit zunehmender Wellenlänge zunimmt. Für die Vermessung des vorderen Augensegments, bei der die Eindringtiefe der Messstrahlung nicht so gross sein muss wie im hinteren Augensegment, kann deshalb eine höhere Wellenlänge A (z.B. 1300 nm) mit höherer optischer Leistung verwendet werden als für die Vermessung des hinteren Augensegments (z.B. 850 nm oder 1060 nm). In Fig. 15 wird die Wellenlänge A punktiert dargestellt, die andere Wellenlänge B wird mit unterbrochenen Strichen dargestellt.

Eine abstimmbare Lichtquelle ALQ-A emittiert eine schmale Spektrallinie (Laserlinie). Über einen 2x2 Faserkoppler FK1-A, eine Optik 01-A, einen Wellenlängen-selektiven Strahlenteiler WLST0 sowie zwei Strahlenteiler ST1 und ST2 wird das Licht in einen Referenz- und in einen Objektarm geleitet. Der Wellenlängen-selektive Strahlenteiler WLST0 ist so beschichtet, dass die Wellenlängen der abstimmbaren Lichtquelle ALQ-A fast vollständig reflektiert werden und die Wellenlängen der abstimmbaren Lichtquelle ALQ-B fast vollständig transmittiert werden. Dadurch werden die beiden Wellenlängen der zwei abstimmbaren Lichtquellen fast verlustfrei vereint. Im Objektarm gelangt das Licht der abstimmbaren Lichtquelle ALQ-A von einem Wellenlängen-selektiven Strahlenteiler WLST1 über einen Polarisationskontroller PK1-A, über einen Spiegel S1, eine Optik 03-A, einen Wellenlängen-selektiven Strahlenteiler WLST2, einen Scanner S, eine Scanoptik SO und einen dritten Strahlenteiler ST3 zum Messobjekt, in diesem Falle zum menschlichen Auge. Die Optik 03-A in Kombination mit der Scanoptik SO fokussiert das Licht der Lichtquelle ALQ-A in das vordere Augensegment. Strahlenteiler ST3 ist ein Wellenlängen-selektiver Strahlenteiler, der das sichtbare Licht zur Kamera K reflektiert, und infrarotes Licht, das normalerweise für die OCT-Lichtquellen verwendet wird, transmittiert. Der Scanner lenkt den Lichtstrahl in ein oder zwei lateralen Dimensionen über die Kornea, von wo der Lichtstrahl ins Auge gebrochen wird. Bei jeder Brechungsindexänderung im Auge wird ein Teil des Lichtes zurückreflektiert. Das zurückreflektierte Licht geht den gleichen Weg zurück bis zum Strahlenteiler ST2. Eine Kamera K nimmt ein 2-dimensionales Bild des Vorderteils des Auges auf, das dem Benutzer auf einem Monitor M zur Verfügung gestellt wird. Das auf dem Monitor dargestellte Kamerabild erlaubt es dem Benutzer, das Messgerät mit Hilfe des Kreuzschlittens KS so vor dem Auge des Patienten zu positionieren, dass die Messung auf das Auge zentriert ist.

Im Referenzarm wird das Licht der Lichtquelle ALQ-A vom Wellenlängen-selektiven Strahlenteiler WLST3 auf den Referenzspiegel RS1 gelenkt. Die Optik 02-A fokussiert den Referenzstrahl auf den Referenzspiegel RS1. Das von diesem Referenzspiegel RS1 zurückreflektierte Licht geht den gleichen Weg zurück bis zum Strahlenteiler ST2. Die Länge des Referenzarms der Lichtquelle ALQ-A ist so ausgelegt, dass dieser Referenzarm das vordere Augensegment ausmisst.

Eine zweite abstimmbare Lichtquelle ALQ-B emittiert eine schmale Spektrallinie (Laserlinie). Über einen 2x2 Faserkoppler FK1-A, einen Wellenlängen-selektiven Strahlenteiler WLST0 sowie zwei Strahlenteiler ST1 und ST2 wird das Licht in einen Referenzarm und in einen Objektarm geleitet. Im Objektarm wird das Licht der abstimmbaren Lichtquelle ALQ-B durch einen Wellenlängen-selektiven Strahlenteiler WLST1 transmittiert, von wo es über einen Polarisationskontroller PK1-B, über einen Wellenlängen-selektiven Strahlenteiler WLST2, über einen Scanner S, eine Scanoptik SO und einen Strahlenteiler ST3 zum Auge gelangt. Die Scanoptik SO fokussiert das Licht der Lichtquelle ALQ-B in das hintere Augensegment.

Im Referenzarm wird das Licht der Lichtquelle ALQ-B vom Wellenlängen-selektiven Strahlenteiler WLST3 auf den Referenzspiegel RS2 gelenkt. Die Optik 02-B fokussiert den

Referenzstrahl auf den Referenzspiegel RS2. Das von diesem Referenzspiegel RS2 zurückreflektierte Licht geht den gleichen Weg zurück bis zum Strahlenteiler ST2. Die Länge des Referenzarms der Lichtquelle ALQ-B ist so ausgelegt, dass dieser Referenzarm das hintere Augensegment ausmisst. Der Pfeil über dem Referenzspiegel RS2 soll andeuten, dass die Ausgangsposition dieses Referenzarms der Referenzspiegelebene RSE2 entspricht, die unmittelbar hinter der längsten zu messenden Achslänge des Auges liegt. Durch das Vorwärtsschieben des Referenzspiegels RS2 wird das Signal der Retina maximiert.

Mit Hilfe der Polarisationskontroller PK1-A, PK2-A, PK1-B und PK2-B bestehend aus den nacheinander platzierten Komponenten Viertelwellenplatte, Halbwellenplatte und Viertelwellenplatte kann die Amplitude der Interferenzsignale maximiert werden.

Im Strahlenteiler ST2 kommt es zur Interferenz des vom Messobjekt und des vom Referenzspiegel reflektierten Lichtes, wobei das Licht der zwei Lichtquellen nur mit sich selber interferieren kann. Am Strahlenteiler ST2 wird das Licht aufgespaltet in einen Teil, der zum Wellenlängenselektiven Strahlenteiler WLST5 geht und einen anderen Teil, der via Strahlenteiler ST1 zum Wellenlängen-selektiven Strahlenteiler WLST4 geht. Die beiden Wellenlängen-selektiven Strahlenteiler WLST4 und WLST5 trennen die Wellenlängen der zwei Lichtquellen und senden das Licht auf die verschiedenen Photodioden PD1-A, PD2-A, PD1-B, PD2-B weiter. Die Interferenzsignale der Photodiode PD1-A und PD2-A haben eine Phasendifferenz von 180°. Diese Phasendifferenz in Kombination mit den zwei entgegengesetzt geschalteten Photodioden PD1-A und PD2-A einer sogenannten balancierten Detektion BD1 ermöglicht es, den Gleichstromanteil der inkohärent überlagerten optischen Signale zu unterdrücken, ohne das Interferenzsignal zu beeinträchtigen. Dasselbe gilt für die Photodioden, die das Licht der Lichtquelle ALQ-B detektieren.

Beide Lichtquellen verfügen über je ein Mach-Zehnder Interferometer, deren Ausgangssignale mit je zwei entgegengesetzt geschalteten Photodioden PD3-A, PD4-A und PD3-B, PD4-B und je einer balancierten Detektion BD2-A und BD2-B gemessen werden. Beide Mach-Zehnder Interferometer bestehen je aus zwei 2x2 Faserkoppler FK4-A und FK5-A beziehungsweise FK4-B und FK5-B. Der Ausgang der balancierten Detektion BD2-A ist die k-Clock der Lichtquelle ALQ-A, k-Clock-A genannt. Der Ausgang der balancierten Detektion BD2-B ist die k-Clock der Lichtquelle ALQ-B, k-Clock-B genannt. Mit Hilfe des Polarisationskontrollers PK4-A wird die Amplitude der auf die zwei Photodioden PD3-A und PD4-A geleiteten Interferenzsignale maximiert. Mit Hilfe des Polarisationskontrollers PK4-B wird die Amplitude der auf die zwei Photodioden PD3-B und PD4-B geleiteten Interferenzsignale maximiert.

Die restlichen Komponenten der Fig. 15 werden schon in der Beschreibung der Fig. 1 erklärt.

### Ausführungsvariante 16

Eine weitere Ausführungsvariante, die zwei unterschiedliche Wellenlängen und zwei unterschiedliche Lichtquellen verwendet, ist in Fig. 16 gezeichnet. In Fig. 16 ist ein Messgerät basierend auf spektraler OCT gezeigt. Im Gegensatz zu dem in Fig. 15 dargestellten Aufbau sind die Lichtquellen LQ-A und LQ-B nicht abstimmbar. Die Lichtquellen LQ-A und LQ-B emittieren ein zeitlich konstantes Spektrum. Weil das Spektrum nicht ändert, braucht diese Ausführung keine k-Clock, insbesondere sind also die in Fig. 15 vorhandenen Mach-Zehnder-Interferometer und die Faserkoppler FK1-A, FK1-B nicht nötig. Zudem ist die balancierte Detektion BD2-A, BD2-B bei spektraler OCT normalerweise nicht vorhanden, deshalb ist sie in Fig. 16 auch nicht vorhanden. Abgesehen vom Quellenarm besteht der einzige Unterschied zu der in Fig. 15 gezeigten Ausführung in der Detektion der Interferenzsignale. Die Interferenzen der Lichtquelle LQ-A und der Lichtquelle LQ-B werden von einem Wellenlängen-selektiven Strahlenteiler WLST4 getrennt und auf zwei unterschiedliche Gitter G-A und G-B geleitet. Die Gitter lenken die verschiedenen Wellenlängen des Spektrums in verschiedene Richtungen ab. Das bedeutet, dass jedes Pixel der Zeilenkameras ZK-A und ZK-B einen bestimmten Ausschnitt aus dem Wellenlängenspektrum der Lichtquelle LQ-A beziehungsweise LQ-B detektiert. Die Optiken 05-A und 05-B fokussieren die verschiedenen Wellenlängen des Spektrums der Lichtquelle LQ-A beziehungsweise LQ-B auf die Zeilenkamera ZK-A beziehungsweise ZK-B. Alle übrigen Komponenten der Fig. 16 sind identisch mit denjenigen in Fig. 15 und sind in der Beschreibung der Fig. 15 erklärt.

### Ausführungsvariante 17

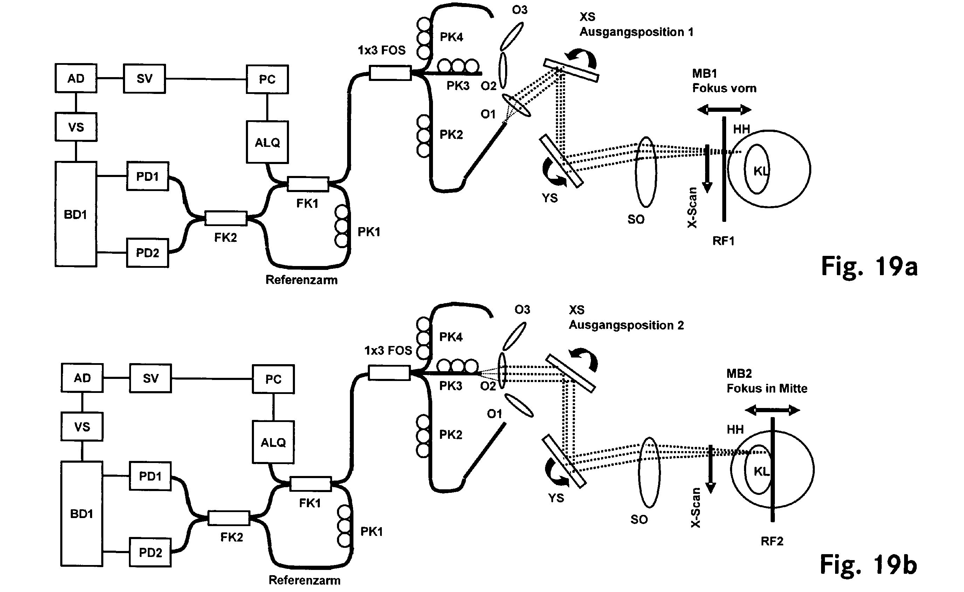

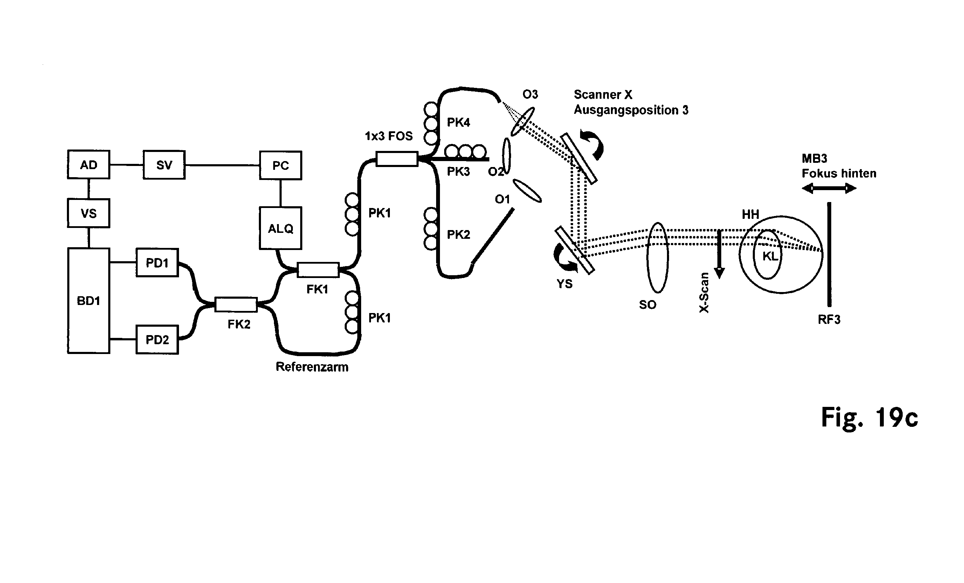

Eine Ausführungsvariante, die synchron den Fokus und die Messdistanz verschiebt, ist in Fig. 19a, 19b und 19c dargestellt. Das Licht einer abstimmbaren Lichtquelle ALQ wird in einen 2x2 Faserkoppler FK1 geleitet. Der Faserkoppler FK1 teilt das Licht in drei Objektarme und einen Referenzarm auf. Der Referenzarm besteht aus einer Monomodefaser und einem Polarisationskontroller PK2. Ein Beispiel eines Polarisationskontrollers ist eine Vorrichtung, in der die Monomodefaser in drei Schlaufen aufgewickelt wird. Jede der drei Schlaufen ist mechanisch kippbar, was erlaubt, dem Licht in der Monomodefaser jeden beliebigen Polarisationszustand aufzuprägen. Der Referenzarm beginnt beim Faserkoppler FK1 und endet beim Faserkoppler FK2. Die optische Länge des Referenzarms bleibt konstant. Die Interferenz des Lichtes des Referenzarms und des Lichtes des verwendeten Objektarms findet im Faserkoppler FK2 statt.

Drei Objektarme werden durch einen faseroptischen 1x3 Schalter FOS erzeugt (englisch: fiberoptic 1x3 Switch). Der faseroptische Schalter FOS leitet das Licht abwechslungsweise in drei unterschiedliche Objektarme. In jedem der drei Objektarme befindet sich je ein Polarisationskontroller PK2, PK3 und PK4 und je eine Optik O1, 02 und 03. Von allen drei Objektarmen gemeinsam verwendet werden ein X-Scanner XS, ein Y-Scanner YS, eine Scanoptik SO. Die drei Objektarme unterscheiden sich in der Brechkraft der drei Optiken O1, 02 und 03 sowie in der optischen Länge gemessen vom faseroptischen 1x3 Schalter FOS bis zur Vorderfläche des Objektes. In Fig. 19a, 19b und 19c ist die Vorderfläche des Objektes die Vorderfläche der Hornhaut. In den Fig. 19a, 19b und 19c sind zur besseren Veranschaulichung der Foki die Messstrahlen durch drei Lichtstrahlen dargestellt.

In Fig. 19a leitet der 1x3 Schalter FOS das Licht in den Objektarm mit der langen Faser. Wenn das Licht durch diesen Objektarm geht, so kommt das Licht, das im vorderen Messbereich MB1 reflektiert wird, zur Interferenz mit dem Licht des Referenzarms. Die Optik O1 in Kombination mit der Scanoptik SO fokussiert das Licht vorzugsweise zwischen der Hornhautvorderfläche HH und der Vorderfläche der kristallinen Linse KL. Die vordere Referenzfläche RF1 ist diejenige Fläche im vorderen Messbereich MB1, die dieselbe optische Länge wie der Referenzarm aufweist. Das bedeutet, dass die Messsensitivität des vorderen Messbereichs MB1 in dieser Fläche RF1 maximal ist. Der X-Scanner XS lenkt das Licht des Objektarms in X-Richtung über das Objekt. Die Bewegungsrichtung des vom X-Scanner XS abgelenkten Messstrahls ist mit dem Pfeil des X-Scan dargestellt. Zu Beginn des X-Scans nimmt der X-Scanner XS die Ausgangsposition 1 an, was dazu führt, dass der Scan in X-Richtung am Rand der Hornhaut beginnt. Der Y-Scanner YS lenkt das Licht des Objektarms in Y-Richtung über das Objekt. Eine Scanoptik SO dient dazu, die Messstrahlen so auf das Auge zu lenken, dass sie im gewünschten Winkel auf die Hornhautvorderfläche auftreffen. Im Normalfall wird man die Scanoptik so wählen, dass die Messstrahlen telezentrisch über das Messobjekt gelenkt werden. Mit Hilfe des Polarisationskontrollers PK2 wird die Polarisation des vom Objekt zurückreflektierten Strahls so eingestellt, dass im Detektionsarm bestehend aus den Photodioden PD1 und PD2, einer balancierten Detektion BD1, einer Verstärkerstufe VS, einem analog-digital Wandler AD und einer Signalverarbeitung SV das höchstmögliche Interferenzsignal detektiert wird. Die Messsignale werden einem Computer PC übergeben, der sie weiterverarbeitet und dem Benutzer als Zahlenwerte oder in bildlicher Form auf einem Monitor zur Verfügung stellt.

In Fig. 19b leitet der 1x3 Schalter FOS das Licht in den Objektarm mit der mittellangen Faser. Die mittellange Faser ist Fig. 19a, 19b und 19c aus Einfachheitsgründen zu kurz dargestellt. Wenn das Licht durch diesen Objektarm geht, so kommt das Licht, das im mittleren Messbereich MB2 reflektiert wird, zur Interferenz mit dem Licht des Referenzarms. Die mittlere Referenzfläche RF2 ist diejenige Fläche im mittlerren Messbereich MB2, die dieselbe optische Länge wie der Referenzarm aufweist. Das bedeutet, dass die Messsensitivität des mittleren Messbereichs MB2 in dieser Fläche RF2 maximal ist. Die Optik 02 in Kombination mit der Scanoptik SO fokussiert das Licht vorzugsweise in der Nähe der Mitte der kristallinen Linse KL. Der X-Scanner XS lenkt das Licht des Objektarms in X-Richtung über das Objekt. Zu Beginn des X-Scans nimmt der X-Scanner XS die Ausgangsposition 2 an, was dazu führt, dass der Scan in X-Richtung am Rand der kristallinen Linse KL beginnt.

In Fig. 19c leitet der 1x3 Schalter FOS das Licht in den Objektarm mit der kurzen Faser. Wenn das Licht durch diesen Objektarm geht, so kommt das Licht, das im hinteren Messbereich MB3 reflektiert wird, zur Interferenz mit dem Licht des Referenzarms. Die Optik 03 in Kombination mit der Scanoptik SO fokussiert das Licht vorzugsweise in der Nähe der hinteren Referenzfläche RF3. Die hintere Referenzfläche RF3 ist diejenige Fläche im hinteren Messbereich MB3, die dieselbe optische Länge wie der Referenzarm aufweist. Das bedeutet, dass die Messsensitivität des hinteren Messbereichs MB3 in dieser Fläche RF3 maximal ist. Der X-Scanner XS lenkt das Licht des Objektarms in X-Richtung über das Objekt. Zu Beginn des X-Scans nimmt der X-Scanner XS die Ausgangsposition 3 an, was dazu führt, dass der Scan in X-Richtung am Rand der Retina beginnt. Für die Messung der Retinasignale kann es in einigen Anwendungen genügen, nur die axiale Distanz der Retina zu messen. In diesem Fall wird der X-Scanner und der Y-Scanner nicht bewegt.

Zur besseren Verständlichkeit sind die Winkel der Ausgangspositionen 1, 2 und 3 in Fig. 19a, 19b und 19c übertrieben unterschiedlich gezeichnet. In einem wirklichen Aufbau wären die Winkel zwischen den optischen Achsen der drei Objektarme deutlich kleiner, damit der Winkelbereich eines handelsüblichen X-Scanners XS (z.B. ein Galvanometerscanner) ausreicht, um die drei Ausgangspositionen 1, 2 und 3 anzufahren.

Die in Fig. 19a, 19b und 19c dargestellte Ausführungsvariante kann auch mit einem 1x2 faseroptischen Schalter oder einem 1x4 faseroptischen Schalter anstelle des 1x3 faseroptischen Schalters betrieben werden. Mit einem 1 x2 faseroptischen Schalter werden zwei Messbereiche und zwei Foki erzeugt, mit einem 1x4 faseroptischen Schalter werden vier Messbereiche und vier Foki erzeugt. Es ist natürlich auch eine Vorrichtung mit einem 1xn faseroptischen Schalter, der n Messbereiche und n Foki erzeugt, machbar.

### Ausführungsvariante 18

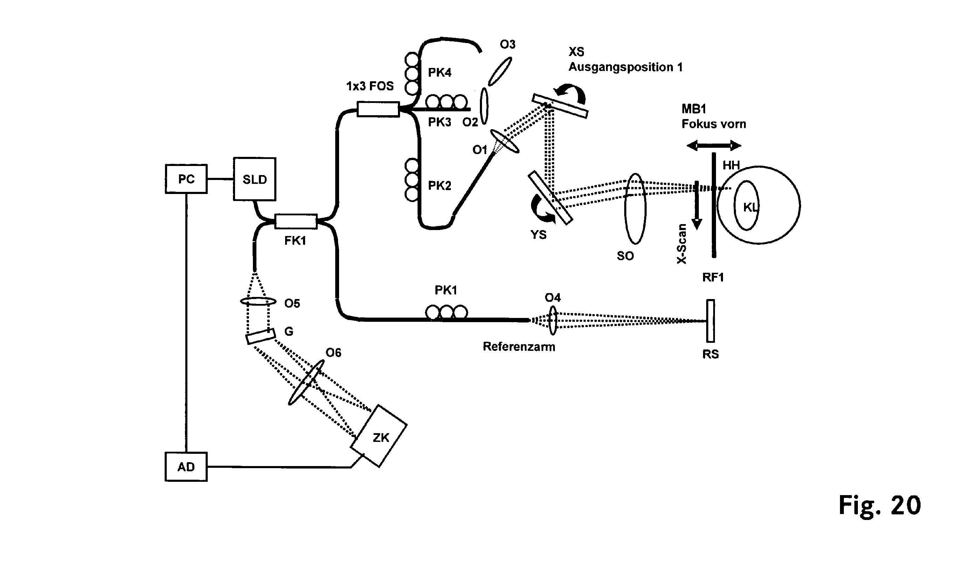

In Fig. 20 wird eine Ausführungsvariante eines spektralen kurzkohärenten Tomographen gezeigt, die die in Fig. 19a, 19b und 19c verwendete Fokus- und Messdistanzschaltung verwendet. Im Gegensatz zu der in Fig. 19a, 19b und 19c dargestellten Ausführungsvariante verwendet die in Fig. 20 gezeigte Ausführung eine Superlumineszenzdiode SLD und ein Spektrometer bestehend aus einem Gitter G, einer Zeilenkamera ZK und den Optiken 05 und 06. Das Licht der Superlumineszenzdiode SLD wird in einen 2x2 Faserkoppler FK1 geleitet. Der Faserkoppler FK1 teilt das Licht in drei Objektarme und einen Referenzarm auf. Der Referenzarm besteht aus einer Monomodefaser, einem Polarisationskontroller PK1, einer Optik 04 und einem Referenzspiegel RS. Die Optik 04 fokussiert den Referenzstrahl auf den Referenzspiegel RS. Der Referenzspiegel RS kann als Option so ausgebildet sein, dass er kontrolliert in Ausbreitungsrichtung des Referenzstrahls verschoben werden kann, um eine oder mehrere Referenzflächen RF1, RF2, und/oder RF3, exakt auf die Distanz der im Objektarm zu messenden Struktur abzustimmen. Das Verschieben des Referenzspiegels RS dient dazu, das Messsignal zu maximieren, was bei sehr niedrigen Messsignalen nötig sein kann.

In Fig. 20 leitet der 1x3 Schalter FOS das Licht in den Objektarm mit der langen Faser. Wenn das Licht durch diesen Objektarm geht, so kommt das Licht, das im vorderen Messbereich MB1 reflektiert wird, zur Interferenz mit dem Licht des Referenzarms. Die Optik O1 in Kombination mit der Scanoptik SO fokussiert das Licht vorzugsweise zwischen der Hornhautvorderfläche HH und der Vorderfläche der kristallinen Linse KL. Die vordere Referenzfläche RF1 ist diejenige Fläche im vorderen Messbereich MB1, die dieselbe optische Länge wie der Referenzarm aufweist. Das bedeutet, dass die Messsensitivität des vorderen Messbereichs MB1 in dieser Fläche RF1 maximal ist. Der X-Scanner XS lenkt das Licht des Objektarms in X-Richtung über das Objekt. Die Bewegungsrichtung des vom X-Scanner XS abgelenkten Messstrahls ist mit dem Pfeil des X-Scan dargestellt. Zu Beginn des X-Scans nimmt der X-Scanner XS die Ausgangsposition 1 an, was dazu führt, dass der Scan in X-Richtung am Rand der Hornhaut beginnt. Der Y-Scanner YS lenkt das Licht des Objektarms in Y-Richtung über das Objekt. Eine Scanoptik SO dient dazu, die Messstrahlen so auf das Auge zu lenken, dass sie im gewünschten Winkel auf die Hornhautvorderfläche auftreffen. Im Normalfall wird man die Scanoptik so wählen, dass die Messstrahlen telezentrisch über das Messobjekt gelenkt werden. Mit Hilfe des Polarisationskontrollers PK2 wird die Polarisation des vom Objekt zurückreflektierten Strahls so eingestellt, dass im Detektionsarm das höchstmögliche Interferenzsignal detektiert wird.

Im Detektionsarm bringt die Optik 05 das aus der Faser austretende Licht kollimiert auf das Gitter G. Das Gitter G beugt die im Spektrum einer breitbandigen Lichtquelle, z.B. einer Superlumineszenzdiode SLD, enthaltenen Wellenlängen in verschiedene Richtungen. Die Optik 06 bildet die sich in der Ausbreitungsrichtung unterscheidenden Wellenlängen örtlich getrennt auf die Zeilenkamera ab. Jedes Pixel der Zeilenkamera detektiert einen schmalen Wellenlängenbereich aus dem Spektrum der Superlumineszenzdiode SLD. Der Ausgang der Zeilenkamera wird in einem Analog-Digital-Wandler AD digitalisiert. Das digitalisierte Signal wird in einem Computer PC Fourier-transformiert. Die FourierTransformation ergibt die Reflexionen des Objektes als Funktion ihres Abstandes von der Referenzfläche RF1. Diese Reflexionen als Funktion des Ortes werden als Intensitätsmuster oder als Datenwerte auf einem Monitor dargestellt. Die Darstellung des Intensitätsmusters kann 1-dimensional (A-Scan), 2-dimensional (B-Scan) oder 3-dimensional (C-Scan) sein.

Die in Fig. 20 gezeigte Ausführungsvariante verwendet einen 1x3 faseroptischen Schalter FOS, was drei Objektarme ergibt, die abwechslungsweise zum Einsatz kommen. Die drei Objektarme ermöglichen die Messung in drei hintereinanderliegenden Messbereichen, wobei der Fokus synchron zur Messbereichsumschaltung in den jeweiligen Messbereich gelegt wird. Aus Einfachheitsgründen wird in Fig. 20 nur der Strahlengang für die Messung des vordersten Augensegments gezeigt. Die Freischaltung des mittleren Messbereichs und des hinteren Messbereichs durch den faseroptischen Schalter FOS ist nicht gezeigt.

Es sind natürlich auch Varianten denkbar, in denen ein 1x2 faseroptischer Schalter, ein 1x4 faseroptischer Schalter oder ein 1xn faseroptischer Schalter eingesetzt werden, die zwei, vier oder n Objektarme erzeugen.

Die in Fig. 20 gezeigte Ausführungsvariante eines spektralen OCT kann auch mit einer anderen als der in Fig. 20 gezeigten Vorrichtung für die Fokus- und Distanzschaltung ausgestattet sein. Denn alle in dieser Patentschrift offenbarten Fokus- und Distanzschaltungen funktionieren sowohl für spektrale OCT-Geräte, die mit einem Spektrometer ausgestattet sind, als auch für OCT-Geräte, die mit einer abstimmbaren Lichtquelle ausgestattet sind SSOCT. Insbesondere kann der Objektarmteil des in Fig. 20 gezeigten spektralen OCT mit einer Vorrichtung für die Fokus- und Distanzschaltung ersetzt werden, die z.B. in den Fig. 8a und 8b, oder 21a und 21b oder 22a, 22b und 22c beschrieben ist.

### Ausführungsvariante 19

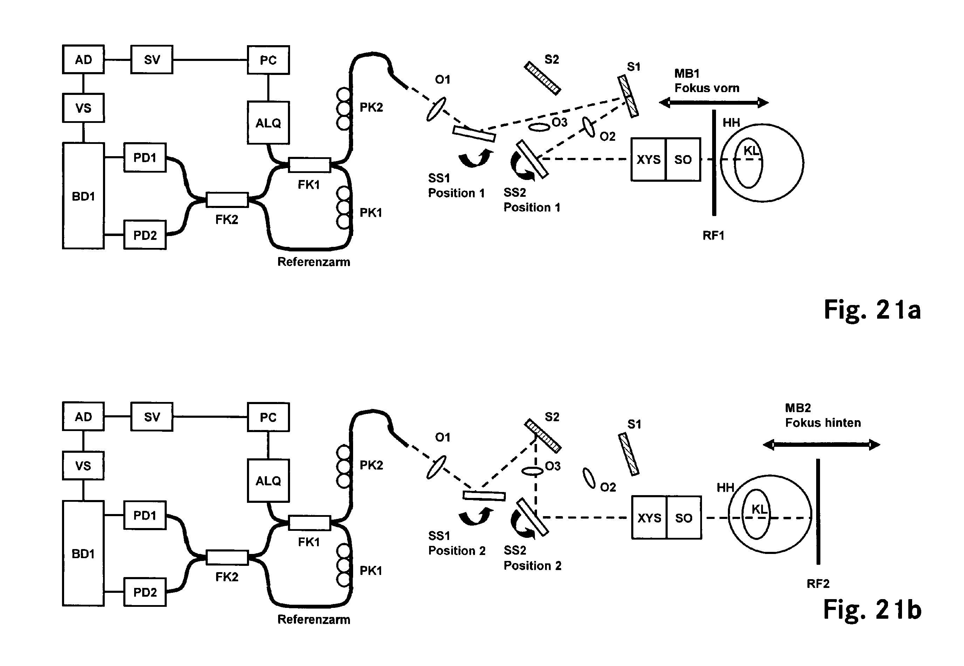

Eine weitere Ausführungsvariante, die synchron den Fokus und die Messdistanz verschiebt, ist in Fig. 21a und 21b dargestellt. Das Licht einer abstimmbaren Lichtquelle ALQ wird in einen 2x2 Faserkoppler FK1 geleitet. Der Faserkoppler FK1 teilt das Licht in die zwei Objektarme und einen Referenzarm auf. Der Referenzarm besteht aus einer Monomodefaser und einem Polarisationskontroller PK1. Der Referenzarm beginnt beim Faserkoppler FK1 und endet beim Faserkoppler FK2. Die Interferenz des Lichtes des Referenzarms und des Lichtes des Objektarms findet im Faserkoppler FK2 statt.

In den Objektarmen befinden sich ein Polarisationskontroller PK2, ein Scannerspiegel 1 SS1, ein Scannerspiegel 2 SS2, ein XY-Scanner XYS, eine Scanoptik SO und die unbeweglichen Spiegel S1, S2 sowie die Optiken O1, 02 und 03. Der Scannerspiegel 1 SS1 leitet das Licht abwechslungsweise in zwei unterschiedliche Objektarme. Die zwei Objektarme unterscheiden sich in der Brechkraft der zwei Optiken 02 und 03 sowie in der optischen Länge gemessen vom Faserkoppler FK1 bis zur Vorderfläche des Objektes. In Fig. 21 a und 21b ist die Vorderfläche des Objektes die Vorderfläche der Hornhaut.

In Fig. 20a lenkt der Scannerspiegel 1 SS1 das Licht in den Objektarm mit dem langen Weg in Luft. Die Ablenkung im richtigen Winkel geschieht nur bei einer bestimmten Position 1 des Scannerspiegels 1 SS1 und des Scannerspiegels SS2. Die Scannerspiegel können beispielsweise Galvanometerspiegel sein. Wenn das Licht durch diesen Objektarm geht, so kommt das Licht, das im vorderen Messbereich MB1 reflektiert wird, zur Interferenz mit dem Licht des Referenzarms. Die Optik O1 in Kombination mit der Optik 02 und der Scanoptik SO fokussiert das Licht vorzugsweise in der Nähe der Vorderfläche der kristallinen Linse KL. Die vordere Referenzfläche RF1 ist diejenige Fläche im vorderen Messbereich MB1, die dieselbe optische Länge wie der Referenzarm aufweist. Das bedeutet, dass die Messsensitivität des vorderen Messbereichs MB1 in dieser Fläche RF1 maximal ist. Der XY-Scanner XYS lenkt das Licht des Objektarms in X-Richtung und Y-Richtung über das Objekt. Eine Scanoptik SO dient dazu, die Messstrahlen so auf das Objekt zu lenken, dass sie im gewünschten Winkel auf die Hornhautvorderfläche treffen. Im Normalfall wird man die Scanoptik so wählen, dass die Messstrahlen telezentrisch über das Messobjekt gelenkt werden. Mit Hilfe der Polarisationskontroller PK1 und PK2 wird die Polarisation des vom Objekt zurückreflektierten Strahls so eingestellt, dass im Detektionsarm bestehend aus den Photodioden PD1 und PD2, einer balancierten Detektion BD1, einer Verstärkerstufe VS, einem analog-digital Wandler AD und einer Signalverarbeitung SV das höchstmögliche Interferenzsignal detektiert wird. Die Messsignale werden einem Computer PC übergeben, der sie weiterverarbeitet und dem Benutzer als Zahlenwerte oder in bildlicher Form zur Verfügung stellt.

In Fig. 21b lenkt der Scannerspiegel 1 SS1 das Licht in den Objektarm mit dem kurzen Weg in Luft. Die Ablenkung im richtigen Winkel geschieht nur bei einer bestimmten Position 2 des Scannerspiegels 1 SS1 und des Scannerspiegels SS2. Wenn das Licht durch diesen Objektarm geht, so kommt das Licht, das im hinteren Messbereich MB2 reflektiert wird, zur Interferenz mit dem Licht des Referenzarms. Die Optik O1 in Kombination mit der Optik 03 und der Scanoptik SO fokussiert das Licht in der Nähe der hinteren Referenzfläche RF2.

### Ausführungsvariante 20

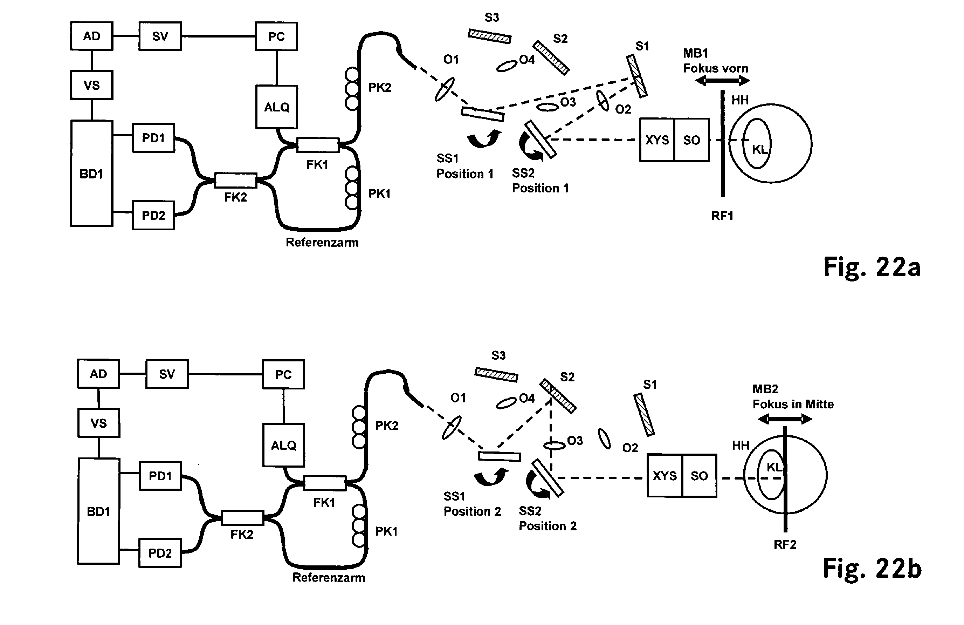

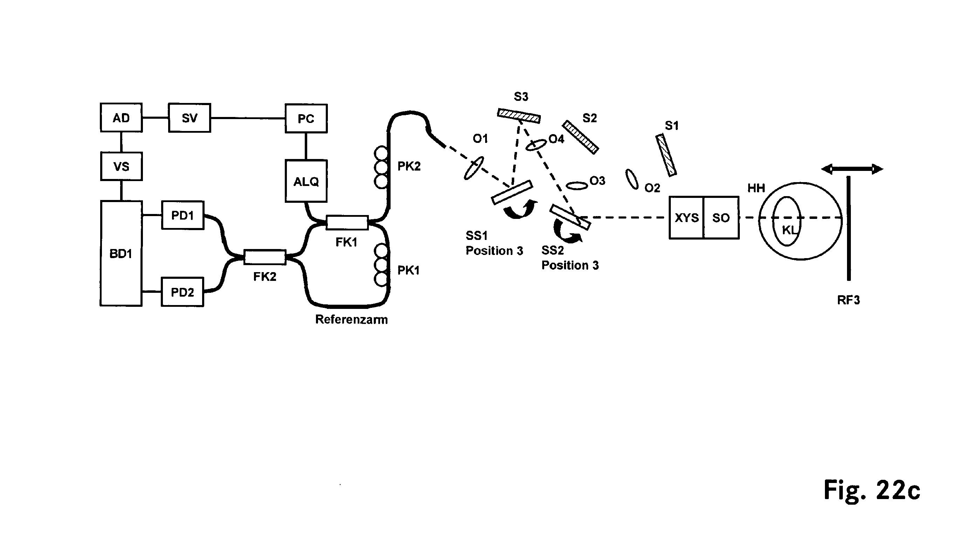

Die in Fig. 21a und 21b dargestellte Ausführungsvariante kann auch mit drei unbeweglichen Spiegeln S1, S2 und S3 oder n unbeweglichen Spiegeln anstelle von zwei unbeweglichen Spiegeln S1 und S2 betrieben werden. Mit drei Spiegeln werden drei Messbereiche und drei Foki erzeugt, mit n Spiegeln werden n Messbereiche und n Foki erzeugt. Eine Ausführungsvariante mit drei Spiegeln S1, S2 und S3 ist in Fig. 22a, 22b und 22c dargestellt.

Zusammenfassend ist festzustellen, dass erfindungsgemäss eine Vorrichtung und ein Verfahren geschaffen wird, welches eine besonders effiziente Messung auch bei Objekten mit grossen Achslängen erlaubt.

## Patentansprüche

1. Vorrichtung zur Ermittlung geometrischer Werte wenigstens eines ersten Bereichs (MB1) und eines vom ersten Bereich (MB1) distanzierten zweiten Bereichs (MB3) eines transparenten oder diffusiven Gegenstands, umfassend einen Kohärenztomographen mit einem Objektarm, einem Referenzarm, einem Detektorarm und einer Lichtquelle (ALQ) zum Emittieren von Licht, **dadurch gekennzeichnet, dass** die Vorrichtung einen durch den Objektarm und/oder den Referenzarm gebildeten ersten Weg einer ersten optischen Weglänge und einen zweiten Weg einer zweiten optischen Weglänge aufweist, entlang welcher sich das von der Lichtquelle (ALQ) emittierte Licht fortpflanzen kann.

2. Vorrichtung nach Anspruch 1, **dadurch gekennzeichnet, dass** der Kohärenztomograph als Frequenzbereichs-OCT, insbesondere als SSOCT oder als spektraler OCT, ausgebildet ist.

3. Vorrichtung nach Anspruch 1 oder 2, **dadurch gekennzeichnet, dass** der geometrische Wert eine Schichtdicke, eine Länge, eine Flächenkrümmung und/oder eine Topographie des Gegenstandes ist.

4. Vorrichtung nach einem der Ansprüche 1 bis 3, **dadurch gekennzeichnet, dass** der Objektarm einen Fokusschalter (FS) umfasst.

5. Vorrichtung nach einem der Ansprüche 1 bis 4, **dadurch gekennzeichnet, dass** der erste Bereich (MB1) ein vorderer Bereich eines Auges, insbesondere die Vorderseite der Kornea, und der zweite Bereich (MB3) ein hinterer Bereich des Auges, insbesondere die Retina sind.

6. Vorrichtung nach einem der Ansprüche 1 bis 5, **dadurch gekennzeichnet, dass** der erste Weg mit der ersten optischen Weglänge durch einen ersten Objektarm und der zweite Weg mit der zweiten optischen Weglänge durch einen zweiten Objektarm gegeben ist.

7. Vorrichtung nach einem der Ansprüche 1 bis 5, **dadurch gekennzeichnet, dass** die erste optische Weglänge durch einen ersten Referenzarm und die zweite optische Weglänge durch einen zweiten Referenzarm gegeben ist.

8. Vorrichtung nach einem der Ansprüche 1 bis 5, **dadurch gekennzeichnet, dass** die erste optische Weglänge durch einen ersten Referenzarm und die zweite optische Weglänge durch einen ersten Objektarm und eine dritte optische Weglänge durch einen zweiten Referenzarm und eine vierte optische Weglänge durch einen zweiten Objektarm gegeben ist.

9. Vorrichtung nach einem der Ansprüche 1 bis 5, **dadurch gekennzeichnet, dass** sie einen Objektarm oder einen Referenzarm mit einem einschwenkbaren und ausschwenkbaren optischen Element umfasst, wobei die erste optische Weglänge bei eingeschwenktem optischem Element gegeben ist und die zweite optische Weglänge bei ausgeschwenktem optischem Element gegeben ist.

10. Vorrichtung nach einem der Ansprüche 1 bis 5, **dadurch gekennzeichnet, dass** sie einen ersten Arm mit einer ersten optischen Weglänge und einen zweiten Arm mit einer zweiten optischen Weglänge aufweist, wobei der erste und der zweite Arm jeweils als Objektarm oder Referenzarm ausgebildet sind, und wobei ein Arm ein optisches Transformationselement (PST1) zum Ändern einer Eigenschaft des Lichtes, insbesondere der Wellenlänge oder der Polarisation, umfasst und wobei der Detektorarm eine optische Trenneinrichtung welche dem optischen Transformationselement entspricht, umfasst.

11. Verfahren zur Ermittlung geometrischer Werte wenigstens eines ersten Bereichs (MB1) und eines vom ersten Bereich (MB1) distanzierten zweiten Bereichs (MB3) eines transparenten oder diffusiven Gegenstands mit einem Kohärenztomographen mit einem Objektarm, einem Referenzarm, einem Detektorarm und einer Lichtquelle (ALQ) zum Emittieren von Licht, **dadurch gekennzeichnet, dass** zur Ermittlung des geometrischen Wertes des ersten Bereichs (MB1) das Licht der Lichtquelle (ALQ) über einen ersten Weg einer ersten optischen Weglänge des Objektarms und/oder des Referenzarms geführt wird und zur Ermittlung des geometrischen Wertes des zweiten Bereichs (MB3) das Licht der Lichtquelle über einen zweiten Weg einer zweiten optischen Weglänge des Objektarms und/oder des Referenzarms geführt wird.

12. Verfahren nach Anspruch 11, **dadurch gekennzeichnet, dass** das Licht nacheinander in einem ersten Objektarm mit dem ersten Weg der ersten optischen Weglänge und in einem zweiten Objektarm mit dem zweiten Weg der zweiten optischen Weglänge geführt ist.

13. Verfahren nach Anspruch 11, **dadurch gekennzeichnet, dass** das Licht in einem ersten Referenzarm mit dem ersten Weg der ersten optischen Weglänge und in einem zweiten Referenzarm mit dem zweiten Weg der zweiten optischen Weglänge geführt ist.

14. Verfahren nach Anspruch 11, **dadurch gekennzeichnet, dass** das Licht nacheinander in einem ersten Referenzarm mit dem ersten Weg der ersten optischen Weglänge und in einem ersten Objektarm mit dem zweiten Weg der zweiten optischen Weglänge, sowie anschliessend in einem zweiten Referenzarm mit einem dritten Weg der dritten optischen Weglänge und einem zweiten Objektarm mit einem vierten Weg der vierten optischen Weglänge geführt ist.

15. Verfahren nach Anspruch 11, **dadurch gekennzeichnet, dass** im Objektarm oder im Referenzarm ein optisches Element eingeschwenkt und ausgeschwenkt wird, so dass sich bei eingeschwenktem optischen Element ein erster Weg der ersten optischen Weglänge und bei ausgeschwenktem optischen Element ein zweiter Weg der zweiten optischen Weglänge einstellt, wobei das Licht nacheinander im ersten Weg und im zweiten Weg geführt ist.

16. Verfahren nach Anspruch 11, **dadurch gekennzeichnet, dass** das Licht gleichzeitig in zwei Arme, insbesondere einem Objektarm, respektive Referenzarm, unterschiedlicher optischer Weglänge geleitet wird, wobei eine optische Eigenschaft des Lichts, insbesondere die Polarisation oder die Wellenlänge, in einem ersten Arm sich von derselben optischen Eigenschaft des zweiten Arms unterscheidet und wobei im Detektorarm das Licht anhand derselben optischen Eigenschaft mittels einer optischen Trenneinrichtung getrennt werden.
